# EUROPEAN PATENT APPLICATION

(11) **EP 4 752 856 A1**
(43) Date of publication of application: **03.06.2026**
(21) Application number: 24216831.8
(22) Date of filing: 02.12.2024
(51) Int. Cl.: G06V 20/69, G06V 10/82, G01N 33/68

(54) **METHOD FOR DETERMINING THE RISK OF DISEASES ASSOCIATED WITH MENTAL HEALTH BY ANALYSING MORPHOLOGICAL CHANGES IN MICROGLIA CELLS**

(71) Applicant: Universidad de los Andes, 7550000 Santiago (CL)
(72) Inventor: LUARTE NAVARRO, Alejandro, Ciudad de Santiago (CL); WYNEKEN HEMPEL, Úrsula, Ciudad de Santiago (CL); LAZCANO GARCÍA, Pablo, Ciudad de Santiago (CL); MUÑOZ EICHLER, Ignacio, Ciudad de Santiago (CL); PRIETO CANCINO, Miguel, Ciudad de Santiago (CL); DEL PIANO COSTÁBAL, José, Ciudad de Santiago (CL)
(74) Representative: Hoffmann Eitle

(57) **Abstract**

The present invention relates to the field of medical diagnosis, and more particularly to methods for detecting the risk of having mental health-related diseases by means of analyzing morphological changes in specific immune cells of the central nervous system, such as microglia, using convolutional neural network-based artificial intelligence.

## Description

### Technical Field of the Invention

The present invention relates to the field of medical diagnosis, and more particularly to methods for detecting mental health-related diseases more accurately by means of analyzing morphological changes in specific immune cells of the central nervous system, such as microglia, using convolutional neural network-based artificial intelligence.

### Background of the Invention

Psychiatric or psychological disorders involve altered thinking, emotions and/or behavior [1]. It is common for these altered aspects to be mild, but when these disorders cause intense distress or interfere with daily living they are considered mental illnesses or mental health disorders, with the effects being temporary or chronic [1]. With respect to the identification of a mental illness, the differentiation of said illness from normal behavior, such as distinguishing a depressive disorder from a normal feeling of sadness about a particular event, is not always clear [1]. Defining a dividing line between having certain personality traits and having a psychiatric disorder becomes a complex challenge based on the presence of symptoms specific to each illness [1]. Furthermore, it is important to emphasize that mental illness-related symptoms are similar among pathologies that constitute different biological and clinical entities and must be correctly diagnosed in order to apply the corresponding treatment, which is specific to each disorder. Cognitive impairment-related mental disorders, which may or may not progress to different types of dementia, gain importance during aging. Diagnosis also has confounding elements and is of primary importance for timely treatment.

Diagnosing mental illnesses objectively presents a significant challenge, particularly given the complexity and subjectivity of many symptoms. In fact, it is estimated that more than one-third of patients with severe psychiatric disorders have been misdiagnosed (39.16%) [19]. In particular, within mood disorders, it is common for both monopolar depression and bipolar disorder to be misdiagnosed, as both present depressive episodes but in the case of bipolar disorder, these episodes alternate with episodes of mania or euphoria. Moreover, in the initial stages of bipolar disorder, a depressive episode may arise. Incorrect treatment of bipolar disorder worsens its prognosis and increases the risk of suicide. The present invention provides new tools for diagnosing mental illnesses objectively.

### Brief Description of the Figures

**Figure 1****:** Exemplary images of cell cultures. (a) Example of an image of the culture (b) Example of an image of the culture (c) Example of an image of the culture for bipolarity disorder. Control patients. Major depressive disorder.
**Figure 2****:** Set of images of the cultures in different color channels. (a) RGB image. (b) Blue channel image. (c) Green channel image. (d) Red channel image.

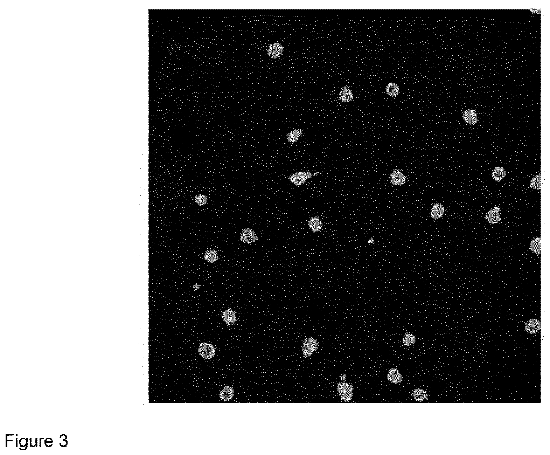

**Figure 3****:** RGB image object segmentation.
**Figure 4****:** Examples of artifacts present within the image of the culture.
**Figure 5****:** Example of cropped images together with correction by means of a Gaussian filter. (a) A cropped image with an artifact present. (b) Correction by means of a Gaussian filter. (c) A cropped image with an artifact present. (d) Correction by means of a Gaussian filter.
**Figure 6****:** Examples of cells not valid for the database.
**Figure 7****:** Cell database distribution.
**Figure 8****:** Examples of a cropped cell image.
**Figure 9****:** Diagram of the process for obtaining the cropped cell image.
**Figure 10****:** Example of randomly created mosaics. (a) Example of a mosaic with 3x3 dimensions (b) Example of a mosaic with 8x8 dimensions.
**Figure 11****:** Diagram of the process for cell classification.
**Figure 12****:** Diagram of the process for mosaic classification.

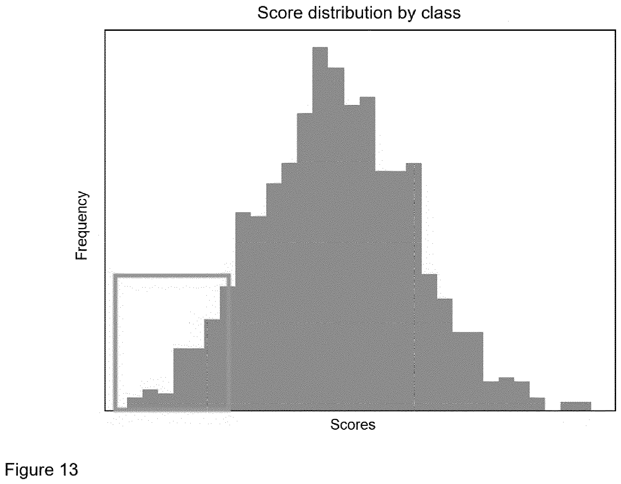

**Figure 13****:** Score distribution for each class.

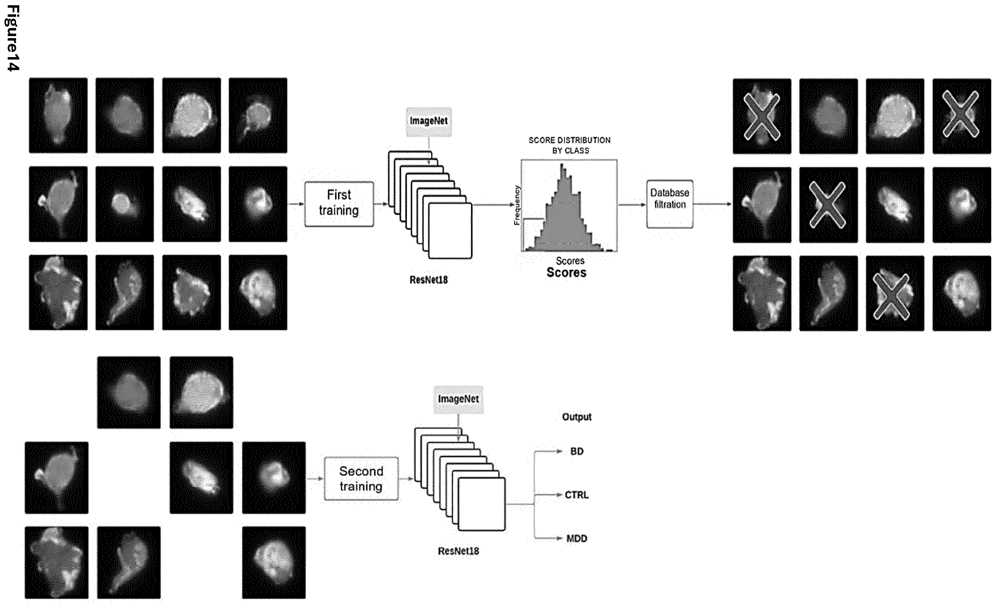

**Figure 14****:** Summary of the process of cell training with a filtered database.

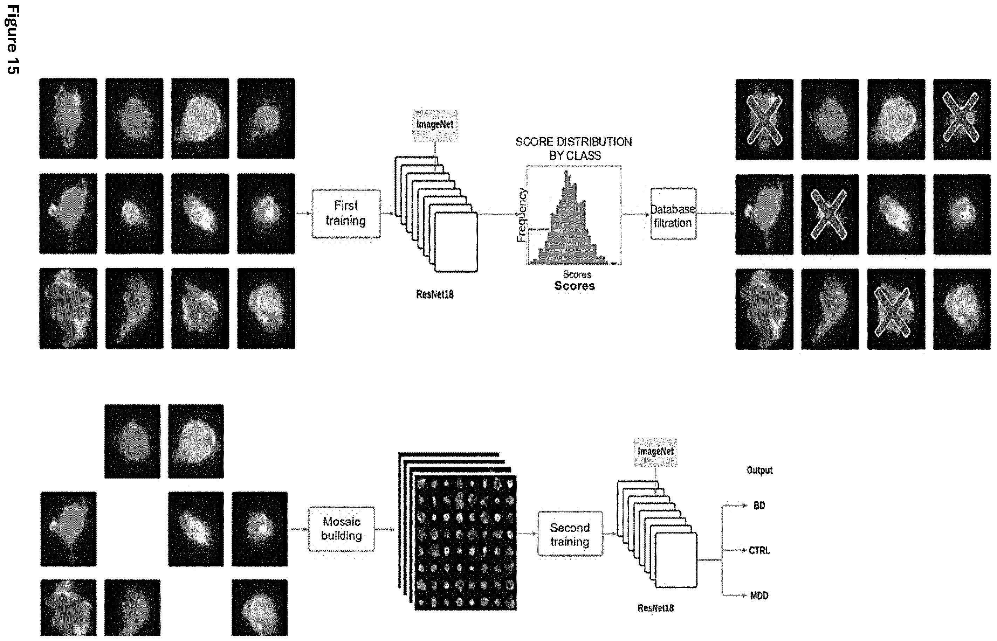

**Figure 15****:** Summary of the process of mosaic training with a filtered database.

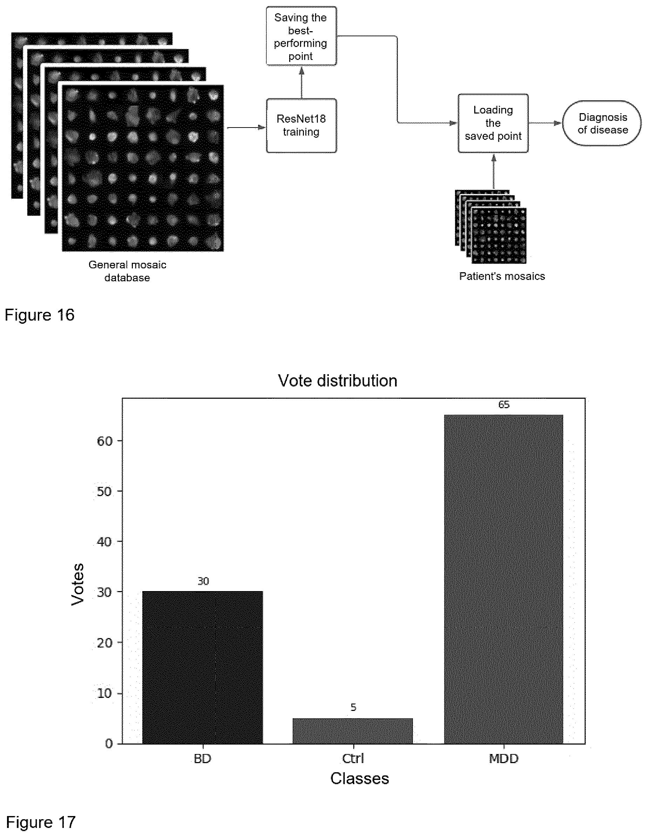

**Figure 16****:** Diagram of the process for the diagnosis of the disease by means of a convolutional network.
**Figure 17****:** Example of votes for a patient.

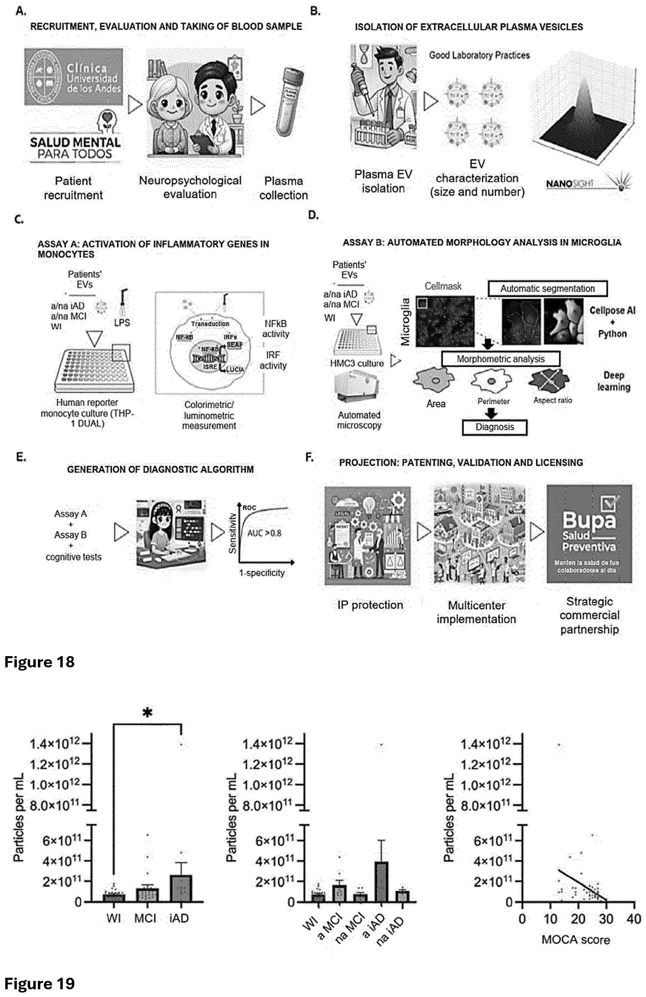

**Figure 18****:** EV- and cellular sensor-based diagnostic platform for the identification of a MCI and iAD subtypes. A. Recruitment of elderly patients from Clinica Universidad de los Andes and the *"Salud Mental para Todos* (Mental Health for All)" program (La Reina). The patients were subjected to a neuropsychological evaluation. Furthermore, an 8 mL blood sample was taken for immediate plasma extraction. B. EVs present in the plasma were isolated by means of standardized methods. The EVs were characterized by size and amount using Nanosight, which allows obtaining quantitative profiles and linking them to the total protein level. C. Assay A evaluated the differential activation of inflammatory pathways in genetically modified human monocytes (THP-1 DUAL). This line stably expresses the secreted alkaline phosphatase (SEAP) and luciferase (Lucia) reporter enzymes, under the control of key inflammatory response elements, such as interferon-stimulated response element (IRSE) and NF-κB-activated promotors, respectively. For this assay, monocytes were stimulated for 24 hours with EVs derived from patients classified into (amnestic or non-amnestic) a iAD and MCI subtypes or healthy subjects (WI, without impairment). LPS at a concentration of 100 ng/ml is used as an inflammation enhancer in the last 4 hours of stimulation with EVs. Differential activation of the NF-κB and IRF pathways is measured through colorimetric and luminometric techniques. D. Human microglia (HMC3) the morphology of which changes differentially in response to patient-derived EVs are used in assay B. EVs are incubated with microglia for 24 hours. Staining and image acquisition in a high-resolution microscope are then performed to conduct an automated morphological analysis by means of Cellpose (AI) in a Python workflow. The morphological response analyzed includes: area, perimeter, aspect ratio, circularity, and roundness, among others. E. Generation of a diagnostic algorithm. F. As a projection, the platform is expected to be protected and validated.
**Figure 19****:** Quantification of the total number of particles present in patient-derived extracellular plasma vesicles. On the left, the graphs show the amount of particles per mL of plasma in the extracellular vesicle sample obtained from WI, MCI, iAD patients. Data is plotted as the mean +/- standard error of N = 23(WI), 22 (MCI), 11 (iAD), *p < 0.05 Kruskal-Wallis. The central panel shows the number of particles in amnestic MCI (a MCI) and non-amnestic MCI (na MCI), initial amnestic dementia (a iAD) and initial non-amnestic dementia (na iAD) conditions. Data is plotted as the mean +/- standard error of N = 23(WI), 8 (na MCI), 8 (a MCI), 5 (na iAD), 6 (a iAD). On the right, linear regression of the data shows an inverse relation between the score obtained in the MOCA (Montreal Cognitive Assessment) test and the amount of particles in the EV fraction obtained from each patient. The slope of the curve is significantly different from 0 (N= 53, p =0.0050).

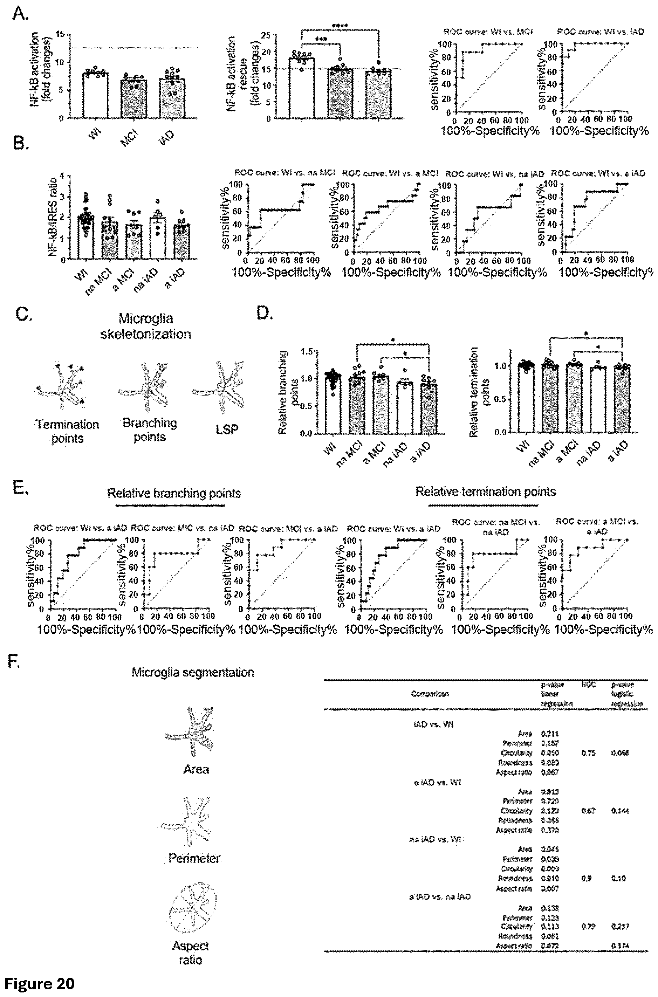

**Figure 20****:** Analysis of inflammation reporter THP-1 response and microglial morphometry in response to patient-derived EVs. A. On the left, there is a comparison of NF-κB activation in THP-1dual monocytes treated with plasma EVs from patients without impairment (WI), with mild cognitive impairment (MCI) and initial dementia (iAD), quantifying the fold changes of alkaline phosphatase activity in comparison to non-stimulated cells. In the central panel, the bar graphs show fold changes in LPS-induced NF-κB activation in monocytes previously treated and not treated (dotted line) with patient-derived plasma EVs. On the right, ROC curves for discriminating between WI and MCI, as well as between WI and iAD, are shown. Data is plotted as the mean +/- standard error of N = 10 (WI), 8 (MCI), 10 (iAD), ***p < 0.001 ****p < 0.0001, one-way ANOVA. B. Ratio of the activation of the NF-κB and IRES pathways in response to LPS (like in A) in monocytes with and without prior treatment with patient-derived EVs. The ROC curves indicate the capacity of the classification algorithm to differentiate between a MCI/na MCI and between a iAD/na iAD. Data is plotted as the mean +/- standard error of N = 37 (WI), 12 (na MCI), 8 (a MCI), 6 (na iAD), 9 (a iAD) patients per condition. C. Diagram of the result of skeletonization, showing branching points and termination points in segmented microglia. D. Comparison of the branching points and termination points in skeletonized microglia after treatment with EVs from the different groups. Data is plotted as the mean +/- standard error of N = 37 (WI), 12 (na MCI), 8 (a MCI), 5 (na iAD), 9 (a iAD) patients, *p < 0.05, one-way ANOVA. E. ROC curves for discriminating between WI and iAD subtypes, using the branching points and the termination points relative to non-EV-stimulated microglial cells as parameters. F. Morphological segmentation of microglia derived from extracellular vesicles. The table on the right shows the p-value of the Wald test for regression, area under the ROC curve and log p-value (patient as a unit). The total number of patients and controls in F is the same as that indicated in Figure A. For all the graphs, each sphere represents the average value of 1000 to 2000 cells for each parameter corresponding to an independent patient.

### Description of the Invention

### - Definitions

**Mental disorder** is defined as a set of symptoms that reveal a clinically relevant disturbance in the cognition, emotional regulation or behavior of a subject, evidencing dysfunction in the underlying psychological, biological or developmental processes that govern mental functioning [2]. These mental disorders are usually linked to substantial distress or disability that has a significant impact on social activities, occupational activities or other important spheres of life. An anticipated or culturally accepted response to common situations of stress or loss, such as the death of a loved one, is not classified as a mental disorder. Likewise, behaviors that deviate socially from the norm (for example, behaviors that are political, religious or sexual in nature) and conflicts primarily between the individual and society are not considered mental disorders, unless such deviance or conflict arises as a consequence of dysfunction in the individual, as previously described [2]. Among mental disorders, mood disorders stand out due to their high prevalence and negative consequences of a misdiagnosis.

**Major depressive disorder (MDD)** is diagnosed when an individual presents a persistently low or depressed mood, anhedonia or decreased interest in pleasurable activities, feelings of guilt or worthlessness, lack of energy, poor concentration, changes in appetite, psychomotor retardation or agitation, sleep disorders or suicidal thoughts [20]. This review addresses the evaluation and management of major depressive disorder, one of the leading causes of disability worldwide, and highlights the role of the interprofessional team [20]. The etiology of major depressive disorder is complex and involves biological, genetic, environmental and psychosocial factors. Traditionally associated with abnormalities in neurotransmitters such as serotonin, norepinephrine and dopamine, recent studies indicate the involvement of more complex neuroregulatory systems and neural circuits [20]. Furthermore, neurotransmitters such as GABA, glutamate and glycine also play a role in depression. Factors such as early stress, hormonal and genetic abnormalities, as well as adverse childhood experiences, contribute to the development of major depressive disorder. Understanding these aspects is crucial to proceed with the evaluation and treatment of this debilitating disorder [20]. More recently, the neurotrophic and neuroinflammation hypotheses have been raised as central factors in the etiopathogenesis of MDD. In terms of treatment, pharmacotherapy has been a key tool, using antidepressants that affect the activity of specific neurotransmitters. However, more recent approaches highlight the importance of psychotherapy and cognitive behavioral intervention as an essential complement in the management of major depressive disorder [20].

**Bipolarity or bipolar disorder (BP).** Bipolar disorder, also known as manic-depressive disorder, is a brain condition that causes significant changes in a person's mood, energy and functional ability. These changes appear in mood episodes, which can be manic/hypomanic (overly happy or irritable mood) or depressive (sad mood). Although people with bipolar disorder also experience periods of neutral mood, the magnitude of these altered emotions is considerable. With suitable treatment, those having bipolar disorder can lead full and productive lives [21]. This disorder affects both brain structure and functionality and can present chronically or episodically. Although everyone experiences normal mood fluctuations, bipolar disorder is characterized by extreme changes that last for days or weeks. Manic episodes appear with an abnormally elevated mood and increased activity, whereas depressive episodes are characterized by sadness and low activity [21]. There are also hypomanic episodes that are less severe but can still affect social and occupational functioning. Bipolar disorder may have a genetic component and usually appears in adolescence or early adulthood, requiring lifelong treatment [21]. Despite the fact that people with bipolar disorder can lead full lives, the disease can affect interpersonal relationships, work and academic performance, and increase the risk of suicide. A thorough understanding of this condition, together with suitable treatment, is essential to improve the quality of life of those having the illness [21].

**Blood plasma.** Plasma is the liquid component of blood in which red blood cells, leukocytes and platelets are suspended [26]. In addition to transporting nutrients, hormones and proteins, plasma also helps regulate body temperature and maintain acid-base balance in the body [27]. Plasma is made up of a variety of substances, including water, mineral salts, proteins, lipids, hormones, enzymes and antibodies [27]. Proteins are one of the most important components of plasma, and are divided into three main categories: albumins, globulins and fibrinogen. Albumins are the most abundant proteins in plasma and perform the function of transporting substances such as hormones, vitamins and minerals [27]. In turn, globulins are proteins that help fight infections and diseases, and fibrinogens are proteins involved in blood coagulation [27].

**Cell morphology.** Cell morphology encompasses the configuration, dimensions and structure of a cell, as well as the arrangement of its organelles. This cellular aspect is commonly used to distinguish and classify cells from one another. However, cell morphology as a whole also provides crucial information about the past and life of each cell [28]. The morphology of each cell is unique; even in a set of cells that is expected to be completely homogeneous, subtle variations may exist between individual cells. Furthermore, cellular processes, such as division and movement, also influence the overall morphology [28]. Various contemporary techniques for diagnosing medical conditions focus on detecting an altered cell morphology. For example, an altered morphology has been identified in certain cells belonging to patients diagnosed with chronic stress [3] or people having autism spectrum disorder [4]. Information about cell morphology serves not only to identify medical conditions, but also as a research tool that facilitates the evaluation of possible treatments and medicinal products [28].

**Microglia.** Microglia are immune cells of the central nervous system (CNS), representing about 10-15% of all glial cells [24]. They are derived from fetal macrophages and are distributed throughout the CNS, exhibiting a branched morphology which allows them to continuously monitor their environment. These cells play crucial roles in the immune defense of the CNS by detecting and removing pathogens, dead cells and cell debris. Furthermore, they are involved in neuroinflammation by releasing inflammatory mediators in response to harmful stimuli. Their role in synaptic remodeling is fundamental, removing weak or damaged synapses and promoting the formation of new neuronal connections [24]. Microglia also provide neuronal support, secreting neurotrophic factors that support neuron growth and survival. Their importance in brain development is evident, as they remove apoptotic cells and regulate neuronal proliferation, ensuring proper brain growth and development [24].

**Extracellular vesicle.** Small extracellular vesicles (sEVs) are tiny membranous structures released by cells of all types. They contain a variety of molecules, including proteins, nucleic acids, lipids and metabolites, which vary according to the physiological/physiopathological state of the cell of origin. These vesicles play a fundamental role in intercellular communication and are involved in various physiological and pathological processes. There are different types of sEVs: exosomes, microvesicles and apoptotic bodies. The fraction obtained in the precipitate corresponding to small extracellular vesicles (30-160 nm) is made up of exosomes, small microvesicles and biological nanoparticles in size. The vesicles contain proteins, mRNA, microRNA and other molecules related to cell function. Microvesicles are larger vesicles (100-1000 nm) containing proteins, cytoplasms and other cellular components. Apoptotic bodies are released by cells dying by apoptosis and contain DNA fragments, histones and other cell death-related proteins [25]. EVs transport molecules between cells, allowing intercellular communication over distances. Furthermore, they remove cell debris, such as misfolded proteins or protein aggregates, from the cytoplasm. Their role in immune response modulation is crucial, activating or inhibiting immune cells. Unfortunately, EVs are also involved in the pathogenesis of various diseases, such as cancer, neurodegenerative diseases and cardiovascular diseases [25].

**Machine Learning.** Machine Learning is a branch of artificial intelligence that mimics the cognitive processes associated with human learning [29]. It involves the use of computational algorithms that are fed by large datasets containing input-output pairs, in order to discern intricate patterns and progressively improve analytical capabilities through iterative learning experiences. In essence, machine learning systems are capable of autonomously refining their predictive or decision-making abilities by means of iteratively modifying algorithms based on the comparison between predicted outputs and known results. The ultimate goal of machine learning is to achieve greater accuracy and efficiency in predicting future results or in generating autonomous recommendations [29].

**Classification problem.** Classification is a supervised machine learning method in which the model attempts to predict the correct label for given input data [29]. In classification, the model is fully trained using the training data and then evaluated with test data before being used to make predictions on new unobserved data [29]. This iterative approach ensures the model's ability to generalize the knowledge acquired during the training phase, allowing it to effectively categorize new instances and thus supporting its effectiveness in real-world applications [29].

**Deep Learning.** Deep Learning is a subcategory of machine learning that focuses on algorithms inspired by brain functioning for knowledge acquisition. Also known as deep structured learning or hierarchical learning, this approach expands the notion of artificial neural networks, scaling them to process substantial amounts of data by means of specific deepening [30]. Through deeper networks, deep learning models can extract data features and learn about these features gradually in each layer, building higher-level knowledge about the data [30]. The technique known as hierarchical feature learning allows these systems to autonomously learn complex features across multiple levels of abstraction, with minimal human intervention [30].

**Convolutional neural networks.** Convolutional neural networks (CNNs) represent a paradigm shift in deep learning, finding inspiration in the structural complexities of the brain's visual cortex. The emulation of receptive fields, where neurons are clustered in overlapping configurations, constitutes the basis of CNNs' ability to analyze and extract features from input data [31]. The architectural essence of CNNs lies in their use of convolution layers which operate by processing input data in overlapping blocks, mimicking the receptive field concept. The progression continues as each convolution layer builds on the features extracted by its predecessor, culminating in the identification of increasingly sophisticated features. This hierarchical feature learning is a fundamental attribute of CNNs, allowing them to automatically discern complex patterns from raw data [31].

**ResNet.** ResNet is a type of neural network architecture that addresses the challenge of training deep networks (with many layers) [32], since conventional deep neural networks become more challenging the more layers the network has given the occurrence of problems such as [32]:
- Vanishing gradient: as the error propagates through the layers in the backpropagation process, gradients become increasingly smaller given the amount of layers, so initial layers may be minimally affected in terms of weight update, slowing down the learning process [32].
- Exploding gradient: Contrary to the preceding problem, given the amount of layers, in certain situations, gradients may become exponentially large as the error propagates.
- Limited representation capability: Deep neural networks may have difficulties with effectively learning and representing complex relationships between input features, which limits their ability to effectively model high-dimensional data [32].
- Increased computational complexity: As more layers are added to the network, the computational complexity and demand for training resources increase significantly, which can make the training process prohibitively expensive in terms of time and resources [32].

ResNet addresses these challenges by introducing shortcut connections that allow information to flow directly through the layers, thereby mitigating the vanishing gradient and providing direct paths for information flow. This strategy has proven to be effective for training extremely deep neural networks, overcoming the challenges associated with network depth and achieving significant advances in accuracy and representation capability [32]. The key concept behind ResNets is the idea of residual learning. Rather than attempting to have each layer of the network directly learn a desired mapping, ResNets allow the layers to learn a residual mapping, i.e., the difference between the desired mapping and the original input. Formally, if the underlying desired mapping is denoted as H(x), ResNets adjust the mapping F(x) := H(x) - x. Then, the original mapping is reformulated as F(x) + x [32]. This formulation is implemented by means of using shortcut connections which allow information to flow directly through the layers, thereby preventing performance degradation problems as additional layers are added. These shortcut connections provide direct paths for information flow, which facilitates the training of extremely deep networks by mitigating the problem of the vanishing gradient [32].

**Building Block ResNet.** The residual learning building block is the basic building unit of the ResNet architecture. This unit is used to build deep neural networks using the residual learning technique [32]. The residual learning building block consists of two convolutional layers and a shortcut connection that skips one or more layers. The first convolutional layer applies one set of filters to the input, while the second convolutional layer applies another set of filters to the output of the first layer. The shortcut connection allows information to flow directly through the layers, thereby preventing performance degradation problems as additional layers are added [32]. The idea behind the residual learning building block is that, by allowing the layers to learn a residual mapping, it becomes easier to optimize the neural network, which allows building deeper and more accurate networks. Furthermore, the shortcut connection provides a direct path for information flow, which facilitates the training of extremely deep networks by mitigating the problem of the vanishing gradient [32]. The residual learning building block can be stacked to build deep neural networks with hundreds or even thousands of layers. These networks have been shown to be highly effective in image recognition tasks, achieving significant advances in accuracy and the ability to train much deeper networks than conventional architectures [32].

**Resnet-18.** ResNet-18 represents a specific architecture within the convolutional neural network paradigm, belonging particularly to the family of residual neural networks (ResNets) [32]. In detail, this architecture is made up of 18 layers, covering convolutional layers, clustering layers and fully-connected layers. Within the framework of the present research, the ResNet-18 architecture has been chosen to be used due to its distinctive feature of being the shallowest version among the various ResNet configurations. This choice is justified based on the ability of this architecture to obtain good results in classification tasks even when relatively modest datasets are available, which is relevant in contexts where the availability of large amounts of data for network tuning and training may be limited. The versatility of ResNet-18 to achieve an effective balance between complexity and performance.

**Convolution layer.** The convolutional layer constitutes a fundamental component within a CNN, representing the place where a significant portion of the computational processes is carried out. This layer, made up of essential elements such as input data, a filter and a feature map, works in a particularly effective manner in image processing. A key component in this layer is the feature detector, also known as a kernel or filter, which goes through the receptive fields of the image, evaluating the presence of specific features by means of convolution [36].

The feature detector, essentially a two-dimensional array of weights representing a segment of the image, is characterized by its ability to vary in size; this filter size determines the dimensions of the receptive field. The filter is applied to a segment of the image, initiating a dot product calculation between the input pixels and the filter [36]. The result of this dot product is then incorporated into an output matrix. Subsequently, the filter undergoes a shift, guided by a specified stride, and the convolution process is executed iteratively until the entire image is covered. The cumulative result of these dot products, which forms the combination of input-filter interactions, is recognized as a feature map, activation map, or a convolved feature [36].

**Rectified linear unit (ReLU) layer.** A rectified linear unit (ReLU) layer performs a threshold operation on each element of the input, in which any value less than zero is set to zero, generally used after convolution [37].

**Batch normalization layer.** Batch normalization applies a transformation that keeps the mean of the output close to 0 and the standard deviation of the output close to 1 [37]. It is important to note that the operation of the layer will vary depending on how the network is being used:
- During training, the layer normalizes its output using the mean and standard deviation of the current batch of inputs [37].
- During evaluation, the layer normalizes its output using a moving average of the mean and standard deviation of the batches observed during training [37].

**Softmax layer.** The Softmax layer is a layer that implements an activation function that tunes the input values into probabilities. The output of a Softmax is a vector with the probabilities of each possible result [37]. The probabilities in the vector add up to one for all possible outputs or classes depending on the input.

**Pooling layer.** Pooling layers, or clustering or subsampling layers, carry out dimensionality reduction by decreasing the number of parameters in the input. Similar to the convolutional layer, the clustering operation shifts a filter along the entire input, but the difference lies in that this filter has no weights [37]. Instead, the kernel applies an aggregation function to the values within the receptive field, populating the output matrix [37]. There are two main types of clustering [37]:
- Maximum clustering: As the filter moves over the input, it selects the pixel with the maximum value to send it to the output matrix. It should be noted that this approach tends to be used more frequently compared to average clustering.
- Average clustering: As the filter moves over the input, it calculates the average value within the receptive field to send it to the output matrix.

Although a lot of information is lost in the clustering layer, it also has several benefits for the CNN. It helps to reduce complexity, improve efficiency and limit the risk of overfitting [37].

**Fully-connected layer.** The term "fully-connected" aptly describes this layer since, in the fully-connected layer, each node in the output layer is directly connected to a node in the previous layer. This layer performs the classification task based on the features extracted through the previous layers and their different filters [37]. While convolution and clustering layers tend to use ReLu functions, fully-connected layers usually employ a Softmax activation function to suitably classify the inputs, generating a probability in the range of 0 to 1 [37].

**Transfer learning.** Transfer Learning constitutes a paradigm in the field of deep learning which uses a model pre-trained for a specific task as a starting point for the initialization of another model intended to carry out a similar task [38]. Updating and retraining a network by means of applying transfer learning proves to be a more efficient and expeditious method compared to comprehensive training from the initial stage. This approach is applicable in several areas, such as image classification, object detection, voice recognition, among other applications [38]. To build a deep learning model, one has the option of training it from scratch or applying transfer learning using a pre-trained model [38]. Creating and training a model from scratch is more effective in highly specific tasks where existing models cannot be utilized. However, this approach usually requires a significant amount of data to obtain accurate results. Furthermore, it is effective in situations where more compact networks can achieve the desired accuracy [38]. Transfer learning is beneficial in tasks for which there are various pre-trained models [38]. For example, many popular convolutional neural networks are trained with the ImageNet dataset, which consists of over 14 million images and thousands of classes. In the case of classifying specific images, such as flowers, with a limited dataset, it is possible to transfer the layers and weights of a pre-trained network, replace the final layers, and proceed to train the model again with the available images [38]. This transfer learning approach emerges as an effective strategy to achieve higher accuracy in a shorter time span.

**Fine-tuning.** Fine-Tuning is a machine learning technique that involves continuing the training of a pre-trained model on a new dataset specific to a particular task [38]. This technique is commonly used in the field of deep learning, where pre-trained models have been pre-trained with large datasets, such as ImageNet, CIFAR-10, MNIST and COCO, to subsequently solve a different problem [38].

The fine-tuning steps are generally as follows [38]:
- Selecting a pre-trained model: a pre-trained model suitable for the specific task is chosen. This pre-trained model has usually been trained with a large, general dataset for a related task.
- Fitting hyperparameters: The hyperparameters of the pre-trained model and the training process are fitted for adaptation to the new dataset and the specific task [38].
- Transferring knowledge: Since the model has been pre-trained with the large dataset, this knowledge is transferred through weight fitting to the new specific problem, i.e., by means of training with the dataset of the task to be solved [38].
- Evaluating performance: The performance of the model fine-tuned on an independent test dataset is evaluated to determine its ability to perform the specific task [38].

**Data augmentation.** Data augmentation is a technique widely used in machine learning and computer vision to increase the amount of training data available for a model [39]. This technique is based on creating new data instances from existing training data by means of applying random transformations to existing images or data. These transformations can include rotations, cropping, scale changes, brightness and contrast changes, among others [39]. The main objective of data augmentation is to improve the capacity of the model to generalize and prevent overfitting by training with a larger and more diverse dataset. By increasing the amount of training data, the capacity of the model to recognize patterns and features in the test data that are not found in the original training data can be improved [39]. Furthermore, data augmentation can also help address the problem of the lack of training data, which is common in many machine learning and computer vision applications. By creating new data instances from the existing training data, the amount of data available to the model can be increased, which can significantly improve its capacity to perform classification and pattern recognition tasks [39].

**Random rotations.** The random rotations technique is a common form of data augmentation used in image processing [39]. It consists of applying random rotations to training images, which involves rotating the image about its center by a random angle. This angle can vary within a predefined range, which introduces variability in the orientation of the training images [39]. Application of random rotations seeks to allow the model to be able to recognize and classify objects regardless of their orientation in the image. This technique is particularly useful in object classification tasks, where the orientation of objects may vary in the input images [39]. By introducing random rotations during training, the variability in the training dataset is increased, which can help the model to generalize better and be more robust to variations in the orientation of the objects in the test images [39].

**Horizontal/vertical mirroring.** Horizontal mirroring involves reflecting the image along a vertical axis, while vertical mirroring involves reflecting the image along a horizontal axis. Horizontal and vertical mirroring are commonly used in object classification tasks, where the orientation of objects may vary in the input images. Application of horizontal and vertical mirroring seeks to allow the model to be able to recognize and classify objects regardless of their orientation in the image [39]. Horizontal and vertical mirroring can be easily implemented in image processing using geometric transformations. In the case of horizontal mirroring, the image can be reflected along the vertical axis by inverting the order of the columns of pixels in the image. In the case of vertical mirroring, the image can be reflected along the horizontal axis by inverting the order of the rows of pixels in the image [39]. By introducing horizontal and vertical mirroring during training, the variability in the training data set is increased, which can help the model to generalize better and be more robust to variations in the orientation of the objects in the test images [39].

**ANOVA test.** Analysis of variance (ANOVA) is a statistical technique used to compare the means of three or more groups and to determine if there are significant differences between them, assuming a Gaussian distribution. The main objective of ANOVA is to evaluate whether the observed differences between groups are due to actual treatment effects or simply to chance [40].

The general process to perform an ANOVA test involves the following steps [40]:
- Formulating hypotheses: two null hypotheses are formulated: the null hypothesis that there are no differences between groups and the alternative hypothesis that at least one group differs from the others [40]..
- Selecting a suitable experimental design: The type of experimental design that fits the data structure, such as one-factor ANOVA, two-factor ANOVA, repeated measures ANOVA, among others, should be determined [40].
- Collecting data: Data needed for the study is collected, ensuring that it complies with the assumptions of ANOVA, such as data normality and variance homogeneity [40].
- Calculating the F statistic: The F statistic, which compares variability between groups with variability within groups, is calculated. A high F value indicates that at least one group differs significantly from the others [40].
- Interpretating results: The calculated F value is compared with a critical F value to determine if there are significant differences between groups. If the calculated F value is greater than the critical value, the null hypothesis is rejected and it is concluded that at least one group differs significantly [40].

**MCI (Mild cognitive impairment):** Mild cognitive impairment is a clinical condition characterized by a decrease in the cognitive abilities of an individual, such as memory, attention or decision-making, that is greater than expected for the age of the subject, but does not significantly interfere with the person's daily activities. It may be an intermediate stage between normal aging and more serious conditions such as dementia.

**Amnestic MCI (a MCI):** An MCI subtype in which the predominant deficits affect memory, with obvious difficulties in remembering recent events or new information. **Non-amnestic MCI (na MCI):** An MCI subtype in which the impairment mainly affects other cognitive abilities, such as executive function, language or visuospatial skills, without significantly affecting memory.

**iAD (Initial amnestic dementia):** This is an early stage of dementia in which memory deficits are the predominant feature, significantly interfering with the daily life of the individual. This subtype is often associated with neurodegenerative diseases such as Alzheimer's in which episodic memory loss is one of the first clinical symptoms.

na iAD (initial non-amnestic dementia): This is an initial form of dementia in which the affected cognitive areas do not predominantly include memory, but involve other abilities such as executive function, reasoning, language or visuospatial perception. This subtype may be related to different neurodegenerative etiologies, such as frontotemporal dementia.

a iAD (initial amnestic dementia): Subtype of initial dementia in which the main symptoms are severe and progressive memory deficits, affecting the ability to remember recent information or past events. This type is mainly associated with Alzheimer's disease or other dementias predominantly in the hippocampus.

Other general definitions are as follows:
**Comprises:**
   Definition: This term is used to indicate that the element or the list of mentioned elements is non-limiting. In other words, it allows the inclusion of other components or features not specifically listed in the claim.
**Consists of:**
   Definition: This term is used to indicate a strict limitation. The list of mentioned elements or features is exhaustive and does not allow the inclusion of other additional components or features.
**About:**
   Definition: This term is used to provide certain flexibility to numerical values or measurements in the description or claim, allowing small variations that one with technical knowledge in the field will be able to understand.
**Preferably:**
   Definition: This term introduces desirable, but non-compulsory, features or configurations to carry out the invention. Generally, it is used to describe optional embodiments.
   **And/or:**
   Definition: This term is used to indicate that one or more of the mentioned elements can be present. It allows an inclusive and flexible interpretation.
**Optionally:**
   Definition: This term indicates that a feature or element is not compulsory but can be included according to specific needs.

### Description

The present invention provides tools for the diagnosis of mental illnesses, specifically age-related diseases and mood disorders. Specifically, the present invention relates to age-related diseases such as cognitive impairment and its more severe form, dementia, and mood disorders such as MDD (major depressive disorder) and BD (bipolarity or bipolar disorder (BP for bipolar disorder)).

Specifically, it can be concluded from Example 1 that the method developed in this invention allows classifying, with a high accuracy, the three classes corresponding to MDD (major depressive disorder), BD (bipolarity or bipolar disorder (BP for bipolar disorder)) and CTRL. To that end a database was created from images originating from microglial cells treated with the plasma of patients classified into the three classes MDD, BD and CTRL. A first database corresponded to individual cell images that were cropped and used to train a CNN.

Another database was then created by building mosaics with individual cell images. The best results were achieved with a mosaic having an 8x8 dimension. The cell images were placed within the mosaic in random positions and rotations. A large number of mosaics can thereby be built from a group of cells belonging to one patient. Several numbers of mosaics were tested per patient, obtaining good results with a number of 50 mosaics per patient. With this strategy, the mosaics were the inputs for the CNN that was trained to separate the three classes MDD, BD and CTRL.

Considering the possibility that not all the cells were altered to enable classifying the disorders MDD, BD or CTRL, a method which allows removing cells of each patient was created. To that end, those cells having a low score in individual classification were filtered. In this way, cells with a higher score were used to create the mosaics, obtaining an improved individual classification.

The classification method obtained good results by using mosaics built with a set of cells belonging to a patient. The best result includes classifying each mosaic corresponding to a patient by adding a vote of the number of mosaics classified by each class. Therefore, the three classes MDD, BD and CTRL can be classified using the method proposed in the present invention.

Moreover, on another note, although there are still no therapies effective for treating mild cognitive impairment (MCI) and its more severe form, dementia, an accurate and timely diagnosis can significantly improve patients' life expectancy and quality of life. However, both conditions are often under-diagnosed, contributing to a significant burden on health systems worldwide. This diagnostic gap is worsen by the lack of accurate, minimally invasive, and cost-effective biomarkers that can support clinical decision making. As illustrated in Example 2, the present invention presents two complementary high-performance platforms designed to distinguish cognitively healthy individuals (WI), individuals with amnestic mild cognitive impairment and individuals with non-amnestic mild cognitive impairment (a MCI/na MCI), and individuals with initial dementia (a iAD/na iAD). The approach of the present invention is based on solid evidence which shows that neurodegenerative conditions leave distinctive inflammatory signatures in extracellular vesicles (EVs) in plasma. To that end, the present invention proposes establishing a new diagnostic method based on the cellular response of sensor cells to patient-derived plasma EVs. First, the morphological response of a human microglia cell line HMC3 (immune cells of the central nervous system) treated with plasma EVs is analyzed in an automated manner. Complementarily, and based on the concept of cellular sensor, a monocyte cell line (THP1-Dual^{™}) stably modified to express and secrete the enzyme alkaline phosphatase (SEAP) under the control of the NfκB promotor and luciferase (LUCIA) under the control of interferon stimulated response elements (IRSE) is used. Activation of these master inflammatory pathways can thereby be detected by means of a simultaneous colorimetric and luminometric reaction for several patients. With this tool, an experiment of the inflammatory response of monocytes treated with EVs from patients compared to a *E.* coli-derived lipopolysaccharide (LPS) challenge is performed, allowing this differential response to discriminate between different diagnostic conditions. During this study, the inventors were capable of recruiting a total of 124 patients from whom the following diagnostic groups were formed: Control without impairment (WI, 69), amnestic mild cognitive impairment (a MCI, 17), non-amnestic mild cognitive impairment (na MCI, 24), initial amnestic dementia (a iAD, 9), initial non-amnestic dementia (na iAD, 5). By means of automated high-resolution microscopy and an analysis that combines artificial intelligence (CellPose program) and Python environment libraries, the inventors automatically draw the cell contour and skeletonize between 1000 and 2000 cells per patient. Based on this data, morphological parameters were extracted to enable discriminating between MCI and iAD subtypes. By means of quantifying the branching and termination points of skeletonized microglia, it is possible to distinguish between patients with a MCI and a iAD with an AUC greater than 0.88. Furthermore, both morphological parameters allow distinguishing between na MCI and iAD with an AUC of 0.77, although amnestic and non-amnestic subtypes are not distinguished within each condition. However, other morphometric parameters such as the circularity of the cell contour allow differentiating between na iAD and a iAD, reaching an AUC of 0.79, highlighting the potential for identifying iAD subtypes. These results allow developing non-invasive, cost-effective diagnostic strategies that integrate each of these strategies or both strategies of cellular sensors for identifying MCI and iAD subtypes.

Therefore, a first aspect of the present invention relates to a method for identifying the risk of a subject having or suffering from an age-related disease or a mood disorder, wherein the method comprises the following steps:
a) exposing specific human immune cells of the central nervous system, preferably microglial cells, *in vitro* to i) a biological sample isolated from the subject which comprises extracellular vesicles and/or ii) a composition which comprises extracellular vesicles isolated from the subject, for a given time, preferably for 1 hour and 72 hours, more preferably for 12 hours and 36 hours, more preferably for about 24 hours;
b) analyzing morphological changes in the cultured microglial cells; and
c) determining the risk of the subject having or suffering from an age-related disease or a mood disorder using a convolutional neural network (CNN) with ResNet architecture trained to recognize morphological patterns indicative of the disease.

Therefore, the methodology of the present invention starts from a biological sample isolated from the subject, preferably a human subject, which must comprise small extracellular vesicles (exosomes and microvesicles or other small-sized nanoparticles). Biological samples can include bodily fluids or tissue extracts. In this sense, the biological sample can be selected from a variety of fluids, such as: blood, plasma, serum, saliva, cerebrospinal fluid, urine, lysed tissue or others. Depending on the nature of the disease to be diagnosed, different types of samples may be preferred, however, preferably in the context of the present invention, the biological sample will be blood plasma.

Moreover, it should be noted that there are various methods for extracting extracellular vesicles useful in the present invention, such as:
a) Ultracentrifugation in density gradients to isolate small extracellular vesicles (more than 100,000 g for several hours).
b) Commercial methods based on chemical precipitations (such as small extracellular vesicle isolation kits).
c) Size exclusion chromatography (SEC) for purifying high-quality extracellular vesicles.

Moreover, in the method of the present invention, the cells cultured *in vitro* must be exposed to the biological sample or to a composition prepared from said sample. This is performed to evaluate how extracellular vesicles affect receiving cells. The exposed cells can be: primary cells derived from human tissue or immortalized cell lines that reproduce relevant features for the disease. Preferably, the cells will be specific human immune cells of the central nervous system, preferably microglial cells. The cells must be maintained in a suitable culture medium to ensure their viability and functional response. Preferably, the cells are microglial cells HMC3, which are a relevant model of the CNS immune system.

The exposure conditions may vary but will preferably be a period of 12 to 48 hours, depending on the rate at which the cells internalize extracellular vesicles at a vesicle concentration preferably between 10⁵ and 10¹² particles/mL.

Analysis of the morphological changes induced in the cells can be performed in terms of, but not limited to:
a) Cell size.
b) Cell shape (elongation, roundness).
c) Cytoplasmic granularity.
d) Distribution of intracellular organelles.
e) Formation of cell aggregates.

Measurements can be performed by means of manual microscopy or automated techniques, such as high content microscopy combined with automated analysis systems, like images obtained by confocal or fluorescence microscopes.

It is also possible to measure other parameters, such as quantitative parameters such as:
a) Cell size measured in square micrometers (µm²).
b) Circularity indices to evaluate cell shape.
c) Organelle distribution viewed by means of specific staining such as DAPI (nuclei) or Mitotracker (mitochondria).

This entire process can be automated by means of using image processing software to generate numerical metrics that describe morphological changes, as well as their possible integration with data capture systems that allow directly feeding neural networks.

Moreover, the method of the present invention uses an artificial intelligence model, particularly a convolutional neural network (CNN), to interpret the data generated and to determine the risk of the subject having the disease. CNN can be based on advanced deep learning architectures, such as ResNet18 or ResNet50. The network must be pre-trained with datasets of cell images labelled as "healthy" or "sick". To carry out said training, an initial high-quality dataset, which includes images of cells exposed to samples from healthy subjects and subjects with the disease, as well as the fine tuning of the neural network by means of learning transfer, using additional images specific of the disease, can be used.

An example of the results generated, which does not limit the present invention, would be a quantitative risk score between 0 and 1, where values close to 1 indicate a high risk of disease, or a display of the result as a heatmap which indicates the areas of the cell images where the network detected relevant patterns.

Optionally, CNN can be executed in a cloud-based infrastructure, allowing remote image analyses and real-time processing. Lastly, integrating morphological results with other clinical biomarkers (such as protein levels or genomic data) may improve diagnostic accuracy and would be achieved by the present invention.

In a preferred embodiment of the present invention, the method identifies the risk of a subject having or suffering from an age-related disease such as cognitive impairment or its more severe form, dementia.

In a preferred embodiment of the present invention, the method identifies the risk of a subject having or suffering from a mood disorder such as MDD (major depressive disorder) or BD (bipolarity or bipolar disorder (BP)).

In a preferred embodiment of the present invention, the method identifies the risk of a subject having or suffering from mild cognitive impairment, preferably amnestic mild cognitive impairment or non-amnestic mild cognitive impairment.

In a preferred embodiment of the present invention, the method identifies the risk of a subject having or suffering from initial amnestic dementia, preferably amnestic initial amnestic dementia or non-amnestic initial amnestic dementia.

In a preferred embodiment of the present invention, the method distinguishes between subjects having or suffering from MCI and iAD.

In a preferred embodiment of the present invention, the method distinguishes between subjects having or suffering from na MCI and iAD.

In a preferred embodiment of the present invention, the method distinguishes between subjects having or suffering from na iAD and a iAD.

In another preferred embodiment of the present invention, optionally in combination with any other embodiment of the present invention, the CNN comprises a ResNet18 or ResNet50 architecture.

In another preferred embodiment of the present invention, optionally in combination with any other embodiment of the present invention, the CNN is trained with a dataset of labelled microglial cell morphology images related to the disease and control samples.

In another preferred embodiment of the present invention, optionally in combination with any other embodiment of the present invention, morphological changes are determined by measuring parameters selected from the group consisting of cell shape, size, granularity, motility, clustering and/or distribution of intracellular organelles.

In another preferred embodiment of the present invention, optionally in combination with any other embodiment of the present invention, the ResNet architecture is pre-trained in a general dataset of images and specifically fitted using a labelled dataset related to the morphological patterns of microglial cells exposed to biological samples from subjects with the disease.

In another preferred embodiment of the present invention, optionally in combination with any other embodiment of the present invention, the biological sample is selected from the group consisting of blood, serum, plasma, saliva, cerebrospinal fluid, urine or tissue lysate, preferably plasma.

In another preferred embodiment of the present invention, optionally in combination with any other embodiment of the present invention, the cultured microglial cells, such as HMC3, are primary cells or cells derived from immortalized cell lines known to exhibit morphological changes in response to biomarkers or toxins associated with the disease.

In another preferred embodiment of the present invention, optionally in combination with any other embodiment of the present invention, the determination of morphological changes comprises high content imaging and automated processing of images to generate quantitative data for analysis by means of the ResNet architecture.

In another preferred embodiment of the present invention, optionally in combination with any other embodiment of the present invention, the method further comprises generating a score of the risk or likelihood that the subject has or suffers from the disease based on the result of the ResNet architecture.

In another preferred embodiment of the present invention, optionally in combination with any other embodiment of the present invention, the ResNet architecture is updated or retained periodically with new labelled datasets in order to improve prediction accuracy and adaptability to variations in the biological samples.

In another preferred embodiment of the present invention, optionally in combination with any other embodiment of the present invention, the risk of disease is determined together with additional clinical parameters or biomarkers from the medical history of the subject in order to improve diagnostic accuracy.

In another preferred embodiment of the present invention, optionally in combination with any other embodiment of the present invention, the method further comprises providing a visual representation or a heatmap of morphological changes in microglial cells in order to help doctors understand the determination performed by the ResNet architecture.

In another preferred embodiment of the present invention, optionally in combination with any other embodiment of the present invention, the ResNet architecture is implemented in a cloud-based platform for the remote analysis and interpretation of cell morphology data.

In another preferred embodiment of the present invention, optionally in combination with any other embodiment of the present invention, morphological changes in microglial cells, such as HMC3, are detected in response to extracellular vesicles, and the ResNet18 or ResNet50 architecture classifies the data based on morphological deviations indicative of the disease.

A second aspect of the present invention relates to a system configured to implement the method of the invention, including any of the preferred embodiments of the first aspect of the invention, wherein the system comprises:
a) an image acquisition module configured to capture high-resolution images of microglial cells exposed to the biological sample or a composition comprising extracellular vesicles;
b) an image processing module configured to analyze morphological changes in the images obtained;
c) an artificial intelligence module which uses a convolutional neural network, preferably based on a ResNet18 or ResNet50 architecture, to classify the morphological changes;
d) a result generation module configured to provide the user with a score of the risk or likelihood of the subject having or developing the disease, and/or optionally a graphic display of the results; and
e) a user interface which allows displaying and downloading the results.

A third aspect of the present invention relates to a computer program stored in a transitory or non-transitory computer-readable medium which, when executed in a computer system, is configured to perform a method for identifying the risk of a subject having or suffering from a disease according to the first aspect of the invention, wherein the program comprises the following instructions:
a) receiving images of microglial cells exposed to a biological sample or a composition comprising extracellular vesicles;
b) processing the received images to detect and quantify morphological changes in the cells;
c) applying a convolutional neural network, preferably based on a ResNet18 or ResNet50 architecture, trained to classify morphological data and to generate a risk score related to the disease; and
d) providing the result of the analysis to the user, preferably by means of a graphic interface or through a cloud-based platform.

Additional aspects of the present invention relate to a transitory or non-transitory computer-readable medium containing a computer program which, when executed in a computer system, implements the method for identifying the risk of a subject having or suffering from a disease according to the first aspect of the invention, wherein the computer program comprises the following instructions:
a) receiving data of the obtained images of microglial cells exposed to a biological sample;
b) performing an automatic segmentation of the cells in the images to identify regions of interest;
c) calculating morphological metrics related to the regions of interest, including size, shape, or intracellular distribution;
d) using a convolutional neural network, preferably ResNet18 or ResNet50, to classify the cells based on the calculated metrics; and
e) generating a result report which includes a risk score and graphical displays of the morphological features.

A computer system comprising:
a) a processor configured to execute a computer program;
b) an input module configured to receive images of microglial cells exposed to a biological sample comprising extracellular vesicles;
c) an analysis module configured to detect morphological changes in the images using a convolutional neural network preferably based on ResNet18 or ResNet50;
d) a result generation module configured to calculate a risk score and provide a visual representation of the morphological changes; and
e) an output module configured to show the risk score and results of the analysis to the user through a graphic interface or a remote platform.

The following examples serve to illustrate the present invention, but do not limit same.

### Examples

### Example 1.

### 1. Methodology

The work performed to achieve the proposed objectives is described in detailed in this example.

### 1.1. Database

This section provides detailed description of the processing of the database corresponding to original cell culture images used to perform cell recognition and cropping, cropping correction, image partitioning according to the number of patients, formation of mosaics with their associated requirements and the transformations used to increase database variability and size.

### 1.1.1. Cell recognition

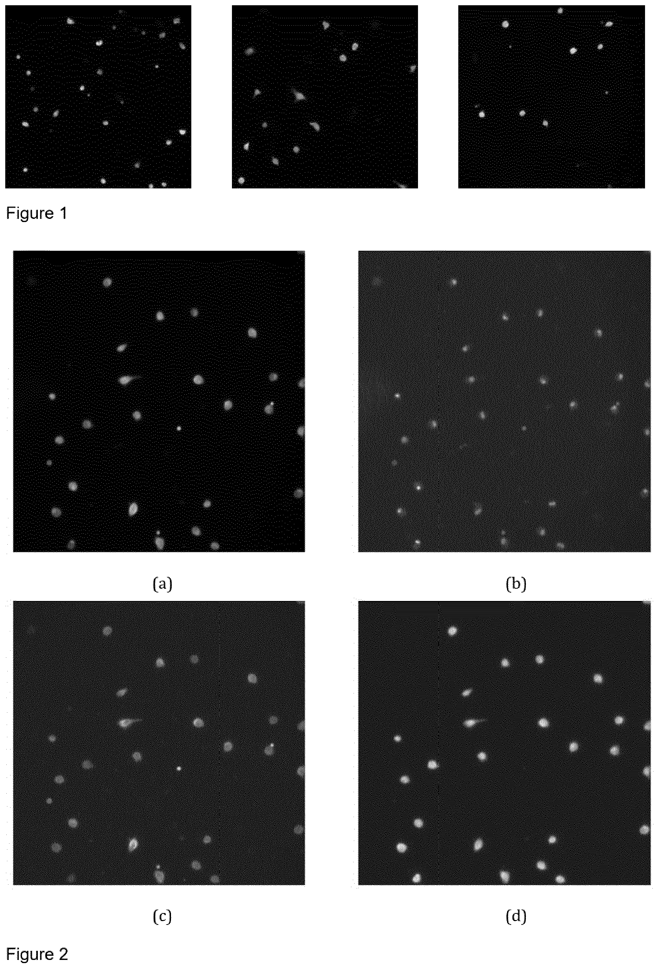

Initially, the database was composed of images of cell cultures with plasma extracted from patients. The way in which these images were obtained was through treatment of sEV-treated microglia, in order to distribute the elements present in the plasma for study, in particular in this work, the cells are the objects of interest (see Figure 1).

For database creation, identification of the objects within the image is necessary to enable segmenting the objects of interest. To that end, identification is performed by means of an edge detector, in order to be able to detect the edges of the objects subsequently, and thereby segment them. The images are represented in three channels, so it is necessary to choose one channel for the edge detection process, for this case the optimal channel is the red channel, because it is the one that best represents the contour of an object in the images.

Therefore, the red channel is used as the preferred channel for edge detection, by applying edge detection and then contour detection, object detection is achieved to enable segmentation for the subsequent cropping of the database. It is important to calibrate the threshold value of image binarization to enable objects to be detected as accurately as possible.

### 1.1.1.1. Binarization threshold calibration

Calibration was performed by comparing data obtained manually with data obtained with automatic binarization given a threshold. The areas of the cells were measured manually, such that data was available for certain cultures and could be compared with the data obtained by means of a sweep performed at different thresholds with binarization. This provides an objective way to find an optimal threshold for object detection.

With the calibration performed, it is possible to detect the objects within the image, where it is now necessary to manage the selection of objects of interest, where criteria of the shape of the objects, as well as object sizes, since most of the cells of interest have a similar size, were implemented. These criteria were implemented by means of an automatic and manual review; this is due to the existence of information such as the cells of interest to be worked on, their location within the image as other data, so it was necessary to corroborate the selected objects to make sure that they were relevant for the database.

### 1.1.2. Individual cell cropping and correction

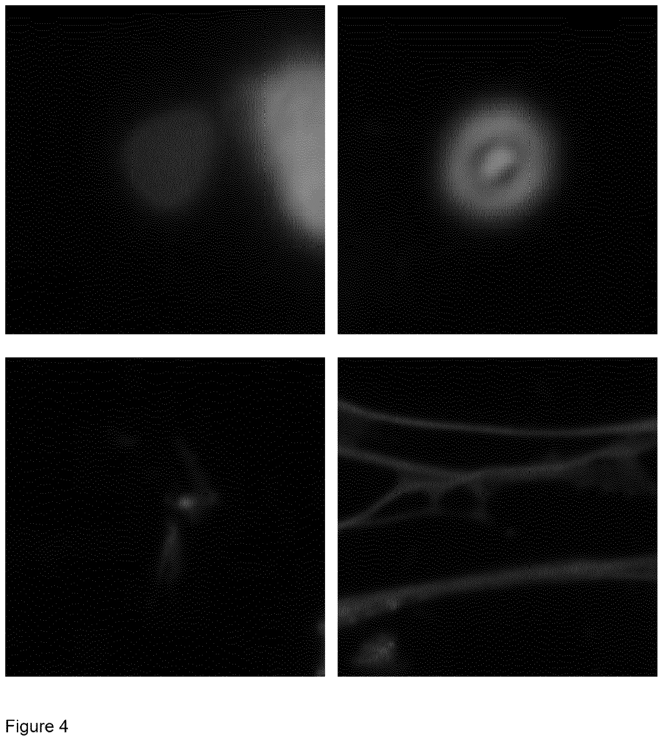

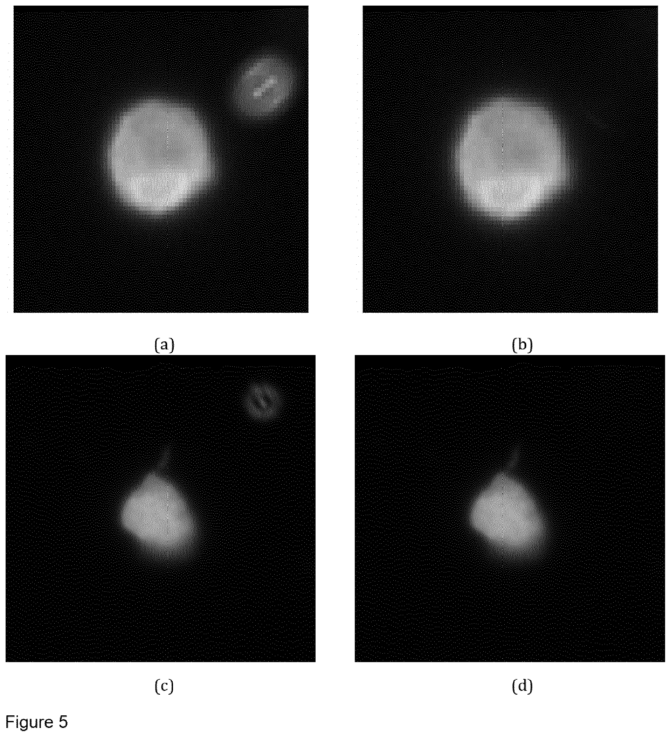

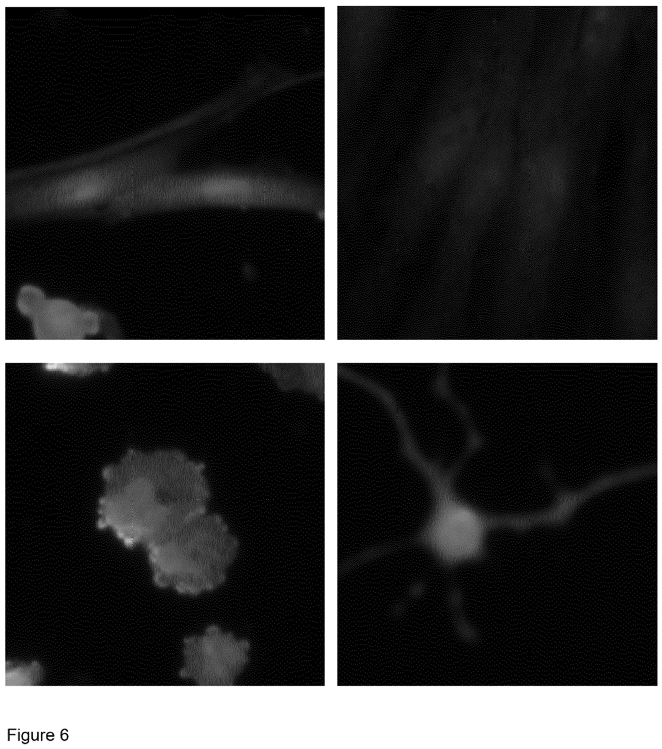

Once the objects of interest are recognized, it is necessary to obtained a cropped image that will belong to the database, the size of the cropped image is 70×70 pixels, such that all the cells fit inside the cropped image, as well as part of the background. When obtaining a cropped image, unwanted artifacts may remain within the extract since, being a cell culture, elements within the blood plasma may be present within the image (see Figure 4). For this reason, all the images were reviewed and a Gaussian filter was suitably applied on the cropped image where there were valid cells so as to correct these unwanted artifacts, so that a square image, where the cell is present in the center with nothing around it, was obtained (see figure 5). There were also times when cell recognition was valid but there were cases where there were indeed cells present in the cropped image, but these cells did not have the characteristics to be considered a valid datum for the database, where there were, for example, two cells within the same cropped image or one cell the membrane of which has broken (see figure 6).

This was performed for the 3 classes, with all their cells respectively, obtaining cropped images to then check for the presence of artifacts for subsequent correction. This process yielded a total of 7319 clipped images, i.e., 7319 cells, as follows:
- 2743 cells corresponding to the BD (*Bipolar Disorder*) class
- 1909 cells corresponding to the CTRL (*Control*) class
- 2667 cells corresponding to the MDD (*Major Depressive Disorder*) class

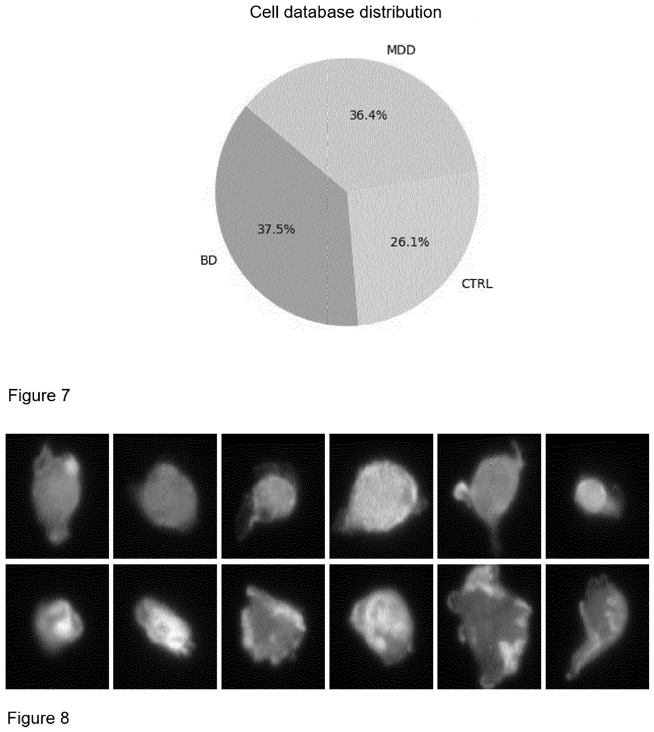

Following this method, the database of the present inventors went from cell culture images to cropped images of the cells of interest, where the class to which it belongs (mental disorder), as well as the patient from which the culture originates (see Figure 8), are identified.

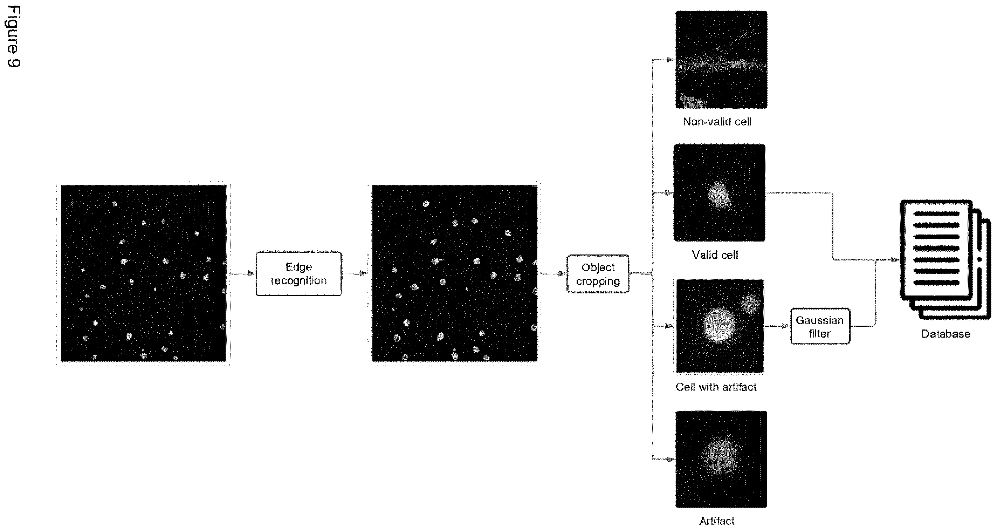

Due to changes in cell morphology, it is important not to modify the real sizes between the cells present in the cropped images, so that it constitutes a factor to be detected subsequently. The summary of the process by which the main database is obtained is thus described in the diagram in Figure 9.

### 1.1.3. Mosaic creation

With the main database of cropped cell images, an additional database corresponding to cell mosaics is created, where square mosaics containing cells are created from the cropped cell images. For mosaic creation, the following requirements had to be met:
- The same mosaic should contain cells of the same class.
- The cells contained in the mosaic must belong to the same patient.

These requirements respond to the need to have mosaics that represent both the class and the patient, so that later, when the classification is performed, it can be performed by the patient, who, in turn, respond to the same class.

Since the mosaics will be formed with the restrictions that the mosaic must contain cells related to the same class and the same patient, the database cannot be partitioned in the conventional manner, where a proportion of the total data is chosen for the training set and the rest for the test set, but must be partitioned to have a fixed number of patients for both groups.

Therefore, the cell database must be partitioned; there are 15 patients for each class, divided into two sets, the training set and the test set, in a proportion of 80% and 20%, respectively; therefore, for each partition, there would be 12 patients for training and 3 patients for testing (see diagram below)

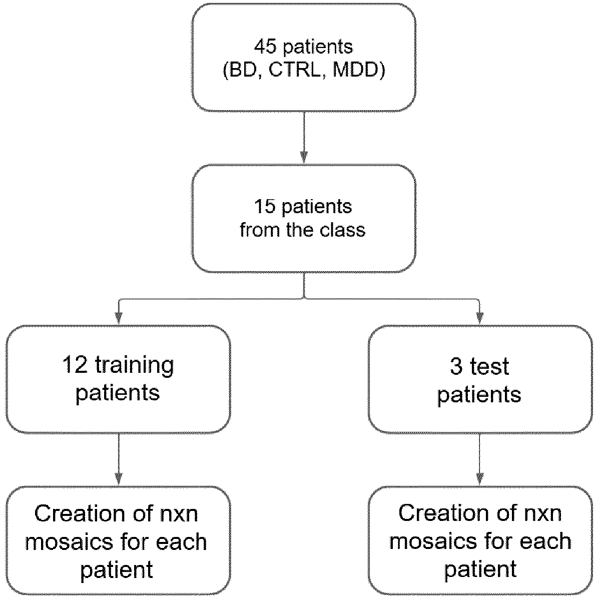

Since patients can be partitioned in different ways, different partitions have been created such that all patients are contained at least once within the test set of a partition.

With this way of partitioning data, mosaics of *n × n* dimensions are created, ensuring that the requirements are achieved constantly. Also, due to the size of the database, to enable creating variability in the mosaics, 3 main methods were used:
- Position: each cell within the mosaic will have a random position.
- Rotation: the cell will have a random rotation.
- Duplication: if necessary, cells could be duplicated including using the above transformations to complete a mosaic.

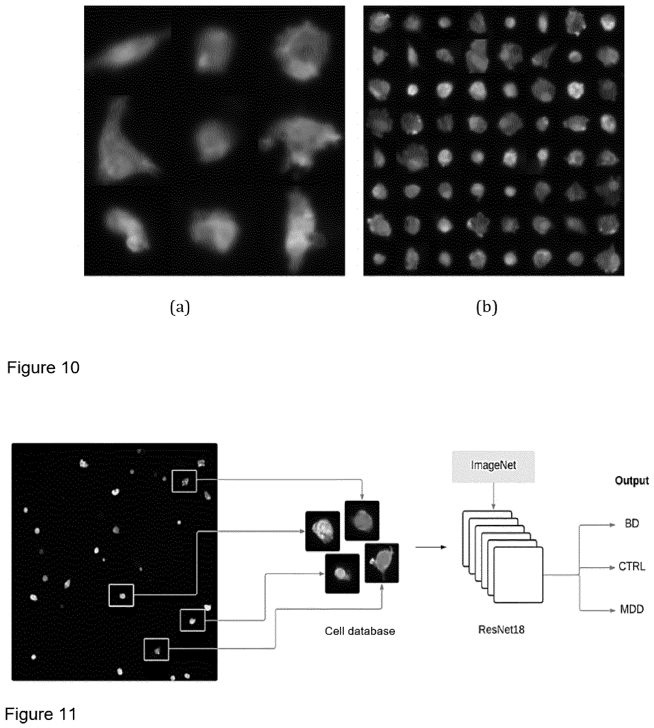

Different *n × n* dimensions were created for the mosaics, starting from 3 *×* 3 to 9 *×* 9 (see Figure 10).

### 1.2. Mental disorder classification

This section addresses the classification challenge associated with the mental disorders presented in this invention. The section is organized into two subsections, where the first subsection focuses on the strategy used for classification using database consisting of individual cells. The preprocessing process applied to the images, as well as the specific conditions implemented during the training of the convolutional network, are examined.

### 1.2.1. Cell classification

Since the intention is to classify mental disorders by means of using a convolutional network, the first experiment was to classify cells directly, and if these cells were differentiable without providing information collected manually, such as cell morphology measured by means of software in each of the cells. Therefore, a choice is made to use the *ResNet* architecture, particularly the *ResNet18* and *ResNet50* architectures were used. Given the *ResNet* architecture, the preprocessing performed was:
- Transformation of the images to tensor.
- Resize of cropped images to 224 *×* 224.

To avoid the random start of network weights, *Transfer learning* was performed as pre-training data obtained by pre-training both architectures with *ImageNet* data was used. Changing the last *ResNet* layer, corresponding to *a fully* connected layer, to the requirements of the cell database classes, training with two classes and training with three classes were performed in particular.

This experiment was the first approach to the possibility of classifying mental disorders by means of convolutional networks. The hyperparameters of cell training are shown in detail in Table 1.

**Table 1: Optimal ResNet-18 training hyperparameters.**

| Parameter | Value |
|---|---|
| Optimizer | SGD |
| Learning rate | 0.001 |
| Momentum | 0.9 |
| Loss function | Cross Entropy Loss |
| Scheduler jumps | 4 epochs |
| Remaining proportion of learning rate | 0.9 |

### 1.2.2. Mosaic classification

The second experiment was to perform classification using the randomly created mosaics as a database, where different tests were performed until the optimal configuration for classification was identified. As mentioned above, the main requirement is that each mosaic is formed by cells belonging to the same patient, such that mosaics associated with the same patient were obtained. The experiments performed were mainly to find the optimal configuration for mosaic formation along with favorable hyperparameters. The following parameters were taken into account for mosaic formation:
- Number of cells per mosaic.
- Number of randomly created mosaics per patient.

Meanwhile for the hyperparameters of the ResNet18 convolutional network the following hyperparameters were mainly taken into account:
- The optimizer used in the training routine, along with the following parameters:
   - Learning rate.
   - The momentum of the optimizer.
- The function for loss calculation in the classification problem.
- Optimal parameters for the scheduler:
   - The jumps between epochs to gradually reduce the learning rate. - The proportion to reduce the learning rate.

With these parameters to be identified, the experiments with the best results had the parameters shown in Table 34.

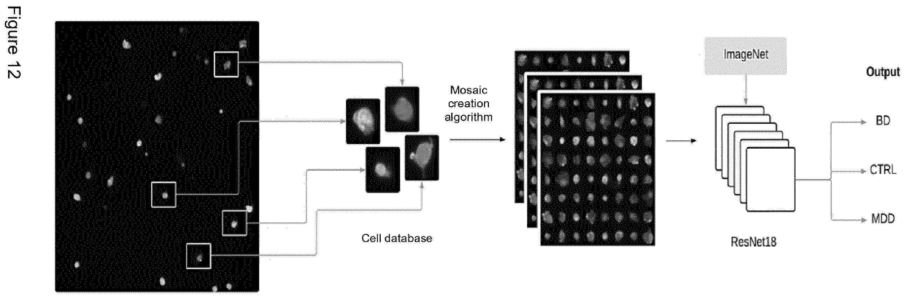

The aforementioned configuration was used for mosaic training experiments, with the same partition types explained above, where there are 15 patients per class, divided into 12 training patients and 3 test patients, having a total of 36 training patients and 9 test patients. The amount of data, given the partitions and the configuration, would be 3600 training mosaics and 900 test mosaics (see Figure 12).

### 1.3. Database filtration

Since at the time of obtaining the sample, the cells must react to the process performed in the extraction and subsequently the treatment already in culture to represent the morphological changes that differentiate each disease, i.e., each class. This is probably not a process that is completely efficient, since not all cells react as they should, but this number is minor with respect to the total number of cells.

To purge these cells from the database the following process was performed:
- General training of the pre-trained model by performing fine-tuning for the cell classification problem, performing multiple iterations finding the point where the model performs optimally without causing overfitting.
- Having found the optimal performance point of the network, this network state is saved.
The set of cells must be run through the network for each partition.
- The results by propagating the set of cells through the network are analyzed, a percentage of the database to be filtered is selected, and for each class the number of corresponding cells is purged based on the score given in the output of the model for each class respectively.
- With the remaining cells, the database is formed again to train the model by performing fine-tuning with this new set of cells obtained.

### 1.3.1. Cell training

Being the base unit of the entire database, the database filtering process in the case of cell database is direct, since it is required to filter the cells the morphology of which did not respond to culture treatment, so it does not represent the class to which it corresponds.

Training is performed with the original database by performing fine-tuning with the pre-loaded ImageNet weights; this training is performed up to the point of optimal performance without performing overfitting, this network state is saved for subsequent use. This network state is loaded by loading the weights, configuring the network in evaluation mode, a propagation of the cells through the network is performed again; thus obtaining a particular distribution of scores given by the network for each cell and class.

With this distribution of scores per class, the objective is to filter out a certain percentage of the part with the lowest scores, which would correspond to cells that do not represent the class in the best way, and therefore did not suitably respond to the culture.

With this filtration of cells with a lower score, the network is re-trained from the weights provided by the pre-training delivered by ImageNet, in order to perform fine-tuning with the remaining cells, obtaining a training with the filtered database. This process was repeated for various percentages, particularly from 5% to 30%.

### 1.3.2. Mosaic training

The database filtration process for the case of mosaic database is identical to the cell filtration process; where first a fine-tuning is performed with the pre-loaded ImageNet weights, an optimal point of the network is chosen, to then propagate the cells through the network to obtain the scores before going through the Softmax layer. With these scores, by class, the distribution that it generates with respect to the cells can be seen and a percentage of the total cells is filtered by evaluating their scores, filtering out the lowest values.

Once the cell database has been filtered, the mosaic is built maintaining the restrictions mentioned in the original process, where the same mosaic must contain cells of the same class and the cells contained in the mosaic must belong to the same patient. Thereafter, the fine-tuning of the network is performed, starting from the loading of ImageNet weights using the mosaics built from the filtered database.

This process is repeated for different filtering percentage values, starting from 5% to 30%.

### 1.4. Voting for the diagnosis of a disorder

The method used to perform an objective diagnosis using the convolutional network was the implementation of a voting system. Taking advantage of the restriction that each mosaic must be formed only by cells from the same patient, it was possible to perform voting for each patient according to his/her mosaics.

Since 100 mosaics were built per patient, and having already trained the network that is now in the evaluation mode, the 100 mosaics of the patient were propagated through the network, checking the classification generated for each mosaic to then add up all the results, observing the winning class and the class with the most accumulated classifications.

Given that the mental disorder is associated with each patient in advance, it is possible to confirm whether the diagnosis generated by the network, given the class with the most mosaic vote, is correct. This voting process was repeated for each patient, and this in each partition.

### 2. Results

The present section presents the results obtained during the execution of the invention. Both quantitative and qualitative findings will be addressed, presenting a detailed evaluation of the collected data and its significance in relation to the objectives of the work. In particular, the results obtained during classification training, as well as the sweeps performed to find the suitable hyperparameters to optimize the results, will be described in detail; to finally present the diagnostic votes given by the network to all the patients in the database.

### 2.1. Binarization threshold sweep

A binarization threshold sweep was performed for different culture images, whereby for each threshold the resulting area of the objects was compared with the manually measured area for each cell. Another important factor is the amount of objects that were detected, in order to filter out as many unwanted artifacts from the object recognition.

**Table 2: Extract of threshold sweep results comparing manually measured areas with areas according to edge detection algorithm.**

| Iteration | Cells | Threshold | Average Units | Average Ratio | Var. Ratio | Std. Ratio |
|---|---|---|---|---|---|---|
| 0 | 10 | 53 | 211.61 | 0.972 | 0.00042 | 0.020 |
| 1 | 10 | 54 | 210.58 | 0.977 | 0.00044 | 0.021 |
| 2 | 10 | 55 | 209.39 | 0.982 | 0.00046 | 0.021 |
| 3 | 10 | 56 | 208.38 | 0.987 | 0.00053 | 0.023 |
| 4 | 10 | 57 | 207.14 | 0.993 | 0.00052 | 0.022 |
| 5 | 10 | 58 | 205.87 | **0.999** | 0.00050 | 0.022 |
| 6 | 10 | 59 | 204.92 | 1.003 | 0.00051 | 0.022 |
| 7 | 10 | 60 | 203.87 | 1.009 | 0.00057 | 0.023 |
| 8 | 10 | 61 | 202.89 | 1.014 | 0.00065 | 0.025 |
| 9 | 10 | 62 | 201.69 | 1.020 | 0.00070 | 0.026 |
| 10 | 10 | 63 | 200.65 | 1.025 | 0.00075 | 0.027 |

The best threshold is chosen according to the average of the ratio between the manually measured area of the objects in the image and those obtained by means of the edge detection algorithm given according to the binarization obtained using the threshold. In other words, the ratio is sought to be the closest to one. An extract of a sweep of different thresholds with their respective metrics is summarized in Table 2.

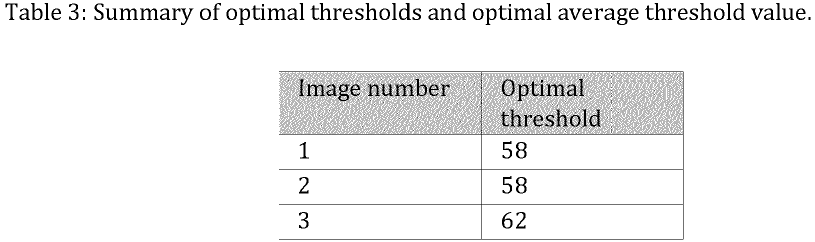

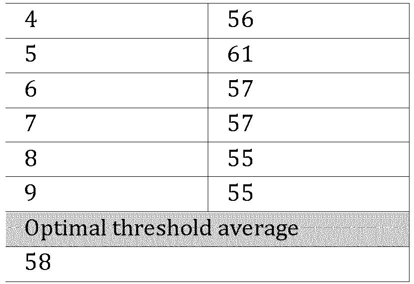

The extract is reviewed and the threshold where the value of the average of the ratio is the closest to one is chosen; obtaining the following thresholds chosen for 9 different images. It is decided that binarization calibration will be performed globally with a pixel value of 58.

### 2.2. Cell classification

Mental disorder classification using individual cells is divided into two experiments, where at first training was performed for the three classes and another training was performed for two classes.

### 2.2.1. 2-class training

For two-class training, combinations of the three classes (Class BD, Class CTRL and Class MDD) were taken. For the two-class case, the aim is to link the ability of the network to differentiate between pairs of classes directly to the morphology of the individual cells. Therefore, training is performed for different partitions of all possible combinations for pairs of classes from the three available classes. Classification is thus performed for pairs of classes in different partitions to verify the classification performance by varying the combination of patients for both the training set and the test set.

**Table 4: Table summarizing the results of 2-class training for database consisting of individual cells. CTRL and MDD classes.**

| 2-class training (CTRL and MDD) | |
|---|---|
| Partition | Test accuracy (%) |
| 1 | 56.48 *±* 0.30 |
| 2 | 67.91 *±* 0.90 |
| 3 | 65.14 *±* 0.63 |
| 4 | 64.73 *±* 0.76 |
| 5 | 69.54 *±* 0.78 |

**Table 5: Table summarizing the results of 2-class training for database consisting of individual cells. BD and MDD classes**

| 2-class training (BD and MDD) | |
|---|---|
| Partition | Test accuracy (%) |
| 1 | 65.57 *±* 1.26 |
| 2 | 63.72 *±* 0.69 |
| 3 | 66.34 *±* 0.42 |
| 4 | 71.13 *±* 0.81 |
| 5 | 70.59 *±* 0.61 |

**Table 6: Table summarizing the results of 2-class training for database consisting of individual cells. BD and CTRL classes**

| 2-class training (BD and CTRL) | |
|---|---|
| Partition | Test accuracy (%) |
| 1 | 77.17 *±* 1.04 |
| 2 | 77.54 *±* 0.43 |
| 3 | 75.91 *±* 0.87 |
| 4 | 76.91 *±* 0.50 |
| 5 | 76.69 *±* 0.53 |

### 2.2.2. 3-class training

For the case of 3-class training, classification was performed for class BD, class CTRL and class MDD simultaneously. This seeks to expose the ability of the network to differentiate the three classes from one another by means of using the morphology of each individual cell for each class. Classification is thus performed for the three classes simultaneously in different partitions to verify the classification performance by varying the combination of patients for both the training set and the test set. The result of what is stated is shown in Table 4.6.

**Table 7: Table summarizing the results of a 3-class training for a database consisting of individual cells. BD, CTRL and MDD classes.**

| 3-class training (BD, CTRL and MDD) | |
|---|---|
| Partition | Test accuracy (%) |
| 1 | 47.09 *±* 0.96 |
| 2 | 51.70 *±* 0.95 |
| 3 | 52.17 *±* 0.89 |
| 4 | 52.00 *±* 0.76 |
| 5 | 54.53 *±* 0.60 |

### 2.3. Mosaic classification

For mental disorder classification by means of cellular mosaics, the results obtained by calibrating the different variables taken into account to perform classification will be described in detail, among the variables taken into account were the characteristics of the mosaics, such as the dimension thereof, as well as the number of mosaics to be created for each patient, as well as variables typical of the network and its training.

### 2.3.1. Dimension of the mosaics

The dimensions that were compared to find the one with the best performance are described in detail, keeping the other variables fixed, a range of length and width from 3x3 to 9x9 was taken, since they are square mosaics. Dimensional leaps are made one by one, which will increase the number of images to be used per mosaic, from the lowest number of 9 cell images per mosaic up to a total of 81 images per mosaic.

**Table 8: Table summarizing the results of classification by means of mosaics with variable dimension.**

| Mosaic dimension | Test accuracy |
|---|---|
| 3x3 | 59.12 *±* 5.48 |
| 4x4 | 56.32 *±* 6.42 |
| 5x5 | 66.67 *±* 6.49 |
| 6x6 | 66.67 *±* 3.98 |
| 7x7 | 65.38 *±* 5.96 |
| 8x8 | 77.57 *±* 3.10 |
| 9x9 | 70.46 *±* 4.95 |

**Table 9: Table 4.8: ANOVA test corresponding to the results presented in Table 8.**

| ANOVA test | | | | | |
|---|---|---|---|---|---|
| Source of variation | Sum of squares | Degrees of freedom | Quadratic means | F | p |
| Between groups | 888.21 | 6 | 148.04 | 5.20 | 0.0052 |
| Within groups | 398.35 | 14 | 28.45 | - | - |

### 2.3.2. Number of mosaics per patient

This variable studies how many random mosaics will be created per patient from the available cells. The dimensions to be studied were narrowed down, in view of the results obtained in the mosaic dimension variation. This number of mosaics to be created per patient determines how many times cell images will have to be repeated or not, depending on the number available for each patient, given that the restriction imposed for the creation of mosaics is that a mosaic to be created must contain cells only and exclusively from the same patient.

**Table 10: Table summarizing the results of classification by means of mosaics, with a variable number of mosaics per patient for a 6x6 dimension.**

| Mosaic dimension | Mosaics per patient | Test accuracy |
|---|---|---|
| 6x6 | 10 | 66.67 *±* 1.25 |
| 6x6 | 50 | 73.50 *±* 1.22 |
| 6x6 | 100 | 68.52 *±* 3.66 |

**Table 11: ANOVA test corresponding to the results presented in Table 10.**

| ANOVA test | | | | | |
|---|---|---|---|---|---|
| Source of variation | Sum of squares | Degrees of freedom | Quadratic means | F | p |
| Between groups | 74.87 | 2 | 37.44 | 6.79 | 0.0287 |
| Within groups | 33.07 | 6 | 5.51 | - | - |

**Table 12: Table summarizing the results of classification by means of mosaics, with a variable number of mosaics per patient for a 7x7 dimension.**

| Mosaic dimension | Mosaics per patient | Test accuracy |
|---|---|---|
| 7x7 | 10 | 65.38 *±* 5.60 |
| 7x7 | 50 | 77.51 *±* 3.26 |
| 7x7 | 100 | 66.85 *±* 3.26 |

**Table 13: ANOVA test corresponding to the results presented in Table 12.**

| ANOVA test | | | | | |
|---|---|---|---|---|---|
| Source of variation | Sum of squares | Degrees of freedom | Quadratic means | F | p |
| Between groups | 262.93 | 2 | 131.47 | 7.49 | 0.0234 |
| Within groups | 105.32 | 6 | 17.55 | - | - |

**Table 14: ANOVA test corresponding to the results presented in Table 13.**

| ANOVA test | | | | | |
|---|---|---|---|---|---|
| Source of variation | Sum of squares | Degrees of freedom | Quadratic means | F | p |
| Between groups | 262.93 | 2 | 131.47 | 7.49 | 0.0234 |
| Within groups | 105.32 | 6 | 17.55 | - | - |

**Table 15: Table summarizing the results of classification by means of mosaics, with a variable number of mosaics per patient for a 8x8 Sum of squares.**

| Mosaic dimension | Mosaics per patient | Test accuracy |
|---|---|---|
| 8x8 | 10 | 78.65 *±* 1.95 |
| 8x8 | 50 | 80.62 *±* 6.77 |
| 8x8 | 100 | 64.07 *±* 4.40 |

**Table 16: ANOVA test corresponding to the results presented in Table 15.**

| ANOVA test | | | | | |
|---|---|---|---|---|---|
| Source of variation | Sum of squares | Degrees of freedom | Quadratic means | F | p |
| Between groups | 490.36 | 2 | 245.18 | 10.64 | 0.0106 |
| Within groups | 138.28 | 6 | 23.05 | - | - |

### 2.3.3. Scheduler: Stepsize and Gamma

The effect of programming a *Scheduler* for the training process was studied, where two main configurations were used, the stepsize which establishes the update of the learning rate value every certain numbers of epoch, as well as the gamma variable which states the change that learning rate value will have. This experiment was performed with a learning rate configuration of 0.1 and with mosaics of 8x8 dimension, with a number of tiles to be created per patient of 50.

**Table 17: Table summarizing the results for the gamma variable, with a fixed stepsize value of 2.**

| gamma | Test accuracy |
|---|---|
| 0.5 | 71.07 *±* 3.12 |
| 0.6 | 75.54 *±* 2.13 |
| 0.7 | 77.68 *±* 2.79 |
| 0.8 | 79.53 *±* 3.16 |
| 0.9 | 75.44 *±* 2.52 |

**Table 18: ANOVA test corresponding to the results presented in Table 17.**

| ANOVA test | | | | | |
|---|---|---|---|---|---|
| Source of variation | Sum of squares | Degrees of freedom | Quadratic means | F | p |
| Between groups | 120.06 | 4 | 30.01 | 3.91 | 0.0366 |
| Within groups | 76.78 | 10 | 7.68 | - | - |

**Table 19: Table summarizing the results for the gamma variable, with a fixed stepsize value of 3.**

| gamma | Test accuracy |
|---|---|
| 0.5 | 78.99 *±* 3.38 |
| 0.6 | 74.62 *±* 4.49 |
| 0.7 | 78.56 *±* 4.35 |
| 0.8 | 76.71 *±* 4.91 |
| 0.9 | 77.13 *±* 3.86 |

**Table 20: ANOVA test corresponding to the results presented in Table 19.**

| ANOVA test | | | | | |
|---|---|---|---|---|---|
| Source of variation | Sum of squares | Degrees of freedom | Quadratic means | F | p |
| Between groups | 179.33 | 4 | 44.83 | 2.50 | 0.0499 |
| Within groups | 1253.21 | 70 | 17.90 | - | - |

### 2.3.4. Learning Rate

The learning rate variable, varying in different values, is studied by performing the classification for each value. This experiment is performed with an 8x8 mosaic dimension, and a number of mosaics to be created per patient of 50. Furthermore, the Scheduler has a *stepsize* of 2 and a gamma of 0.8.

**Table 21: Table summarizing the results of classification by means of mosaics, with a learning rate variable.**

| Learning rate | Test accuracy |
|---|---|
| 0.01 | 80.74*±* 4.38 |
| 0.009 | 76.04 *±* 3.46 |
| 0.008 | 72.34 *±* 2.40 |
| 0.0075 | 83.74 *±* 3.98 |
| 0.006 | 75.43 *±* 2.63 |
| 0.005 | 70.23 *±* 2.42 |

**Table 22: ANOVA test corresponding to the results presented in Table 21.**

| ANOVA test | | | | | |
|---|---|---|---|---|---|
| Source of variation | Sum of squares | Degrees of freedom | Quadratic means | F | p |
| Between groups | 384.99 | 5 | 76.99 | 7.02 | 0.0028 |
| Within groups | 131.56 | 12 | 10.96 | - | - |

### 2.3.5. Fine-tuning

The application of fine-tuning in training is studied and pre-training with *ImageNet* in ResNet-18 is used. All the experiments are performed again using this technique, obtaining results similar to all the previous tests for the case of mosaics. All the subsequent tests have an 8x8 mosaic dimension and a number of mosaics to be created per patient of 50.

**Table 23: Comparison of the use of fine-tuning in training compared to standard training with random initial weights of a random partition.**

| Training mode | Test accuracy |
|---|---|
| Standard | 79.53 *±* 3.16 |
| Fine-tuning | 80.57 *±* 0.93 |

With fine-tuning, the learning rate values are scanned again with the *scheduler* configured with a *stepsize* of 2 and a *gamma* of 0.8.

**Table 24: Table summarizing the results for learning rate values with a stepsize 2 and a gamma of 0.8 using fine-tuning.**

| Learning rate | Test accuracy |
|---|---|
| 0.01 | 78.75 *±* 3.24 |
| 0.009 | 77.59 *±* 1.65 |
| 0.008 | 83.34 *±* 0.33 |
| 0.007 | 78.57 *±* 1.32 |
| 0.006 | 77.91 *±* 1.28 |
| 0.005 | 79.07 *±* 3.18 |

**Table 25: ANOVA test corresponding to the results presented in Table 24.**

| ANOVA test | | | | | |
|---|---|---|---|---|---|
| Source of variation | Sum of squares | Degrees of freedom | Quadratic means | F | p |
| Between groups | 110.06 | 5 | 22.01 | 4.92 | 0.0030 |
| Within groups | 107.29 | 24 | 4.47 | - | - |

Tests are performed again by varying the number of mosaics per patient.

**Table 26: Table summarizing the results for different numbers of mosaics per patient, using fine-tuning.**

| Number of mosaics | Test accuracy |
|---|---|
| 30 | 79.15 *±* 1.92 |
| 40 | 76.84 *±* 2.26 |
| 50 | 83.34 *±* 0.33 |
| 60 | 80.92 *±* 3.33 |
| 70 | 81.88 *±* 1.94 |

**Table 27: ANOVA test corresponding to the results presented in Table 26.**

| ANOVA test | | | | | |
|---|---|---|---|---|---|
| Source of variation | Sum of squares | Degrees of freedom | Quadratic means | F | p |
| Between groups | 126.69 | 4 | 31.67 | 6.67 | 0.0014 |
| Within groups | 95.02 | 20 | 4.75 | - | - |

Random partitions are performed with different combinations of patients to confirm the robustness of the model. These partitions are created from random combinations of patients for training and test, maintaining the restriction of 12 training patients and 3 test patients.

**Table 28: Table summarizing the results of the model with different partitions of training and test patients, using fine-tuning.**

| Additional partitions | Test accuracy |
|---|---|
| 1 | 76.00 *±* 1.26 |
| 2 | 87.62 *±* 0.96 |
| 3 | 89.00 *±* 1.91 |
| 4 | 86.90 *±* 3.90 |
| 5 | 83.34 *±* 0.33 |

### 2.4. Database filtration

Database filtration and retraining are performed using the preceding methodology. The results obtained for this methodology both for individual mosaic and for voting will be shown.

**Table 29: Table summarizing the results of the percentages of filtration and their respective result for individual mosaic training.**

| % of filtration | Test accuracy |
|---|---|
| Unfiltered | 84.86 *±* 0.0312 |
| 5% | 86.66 *±* 0.1456 |
| 10% | 86.62 *±* 0.2987 |
| 15% | 84.62 *±* 0.4234 |
| 20% | 83.49 *±* 0.5175 |
| 25% | 84.10 *±* 0.6026 |
| 30% | 81.57 *±* 0.6783 |

**Table 30: ANOVA test corresponding to the results presented in Table 29.**

| ANOVA test | | | | | |
|---|---|---|---|---|---|
| Source of variation | Sum of squares | Degrees of freedom | Quadratic means | F | p |
| Between groups | 57.13 | 6 | 9.52 | 48.24 | 1.4752e-08 |
| Within groups | 2.76 | 14 | 0.20 | - | - |

**Table 31: Table summarizing the results of the percentages of filtration and their respective result for voting.**

| % of filtration | Voting accuracy |
|---|---|
| base | 91% |
| 5% | 89% |
| 10% | 91% |
| 15% | 91% |
| 20% | 84% |
| 25% | 89% |
| 30% | 84% |

### 2.5. Voting

The following section provides detail of the results obtained by means of the voting methodology. There are two sets of results, each with specific restrictions. For the first set of results the following restrictions were established:
- All the patients appeared at least once in the test set.
- Use of the minimum number of partitions.

This is why the first set of results is made up of 5 partitions, since this way the minimum number of partitions is obtained with all the patients appearing at least once in the test set. This is presented in Table 32.

**Table 32: Diagnosis voting results for the first set of results with restrictions.**

| Partition | Correct diagnosis | Incorrect diagnosis | Percentage of correct diagnoses |
|---|---|---|---|
| 1 | 8 | 1 | 88.89% |
| 2 | 8 | 1 | 88.89% |
| 3 | 9 | 0 | 100% |
| 4 | 9 | 0 | 100% |
| 5 | 9 | 0 | 100% |
| | Total | | 95.56% |

Where the two erroneous patients in the set of results correspond to one patient in the CTRL class and one patient in the MDD class.

For the second set of results, the way the different partitions were built was random. This was intended for building various cases of patient combinations and evaluating the performance of the method in different scenarios. 25 random combinations of patients were performed for the training set and the test set, generating a total of 225 diagnostic votes. The results are presented in Table 33.

**Table 33: Table summarizing votes for the voting methodology.**

| Overall vote summary | | |
|---|---|---|
| Combinations | 25 | |
| Votes | 225 | |
| Correct votes | 219 | |
| Erroneous votes | 6 | 4 MDD |
| | | 2 CTRL |
| Accuracy | 97.33% | |

**Table 34: Optimal ResNet-18 training hyperparameters.**

| Parameter | Value |
|---|---|
| Mosaic dimension | 8x8 |
| Mosaics per patient | 100 |
| Optimizer | SGD |
| Learning rate | 0.008 |
| Momentum | 0.9 |
| Loss function | Cross Entropy Loss |
| Scheduler jumps | 2 epochs |
| Remaining proportion of learning rate | 0.8 |

### Example 2.

### 1. General description of the experimental strategy

Elderly patients (>60 years) were recruited and subjected to the withdrawal of a small blood sample to obtain plasma and a detailed neuropsychological evaluation (Figure 18A). Subsequently, plasma EVs were isolated and characterized (Figure 18B), which were analyzed by means of cellular sensors. To that end, cellular sensors were used with two approaches. The first strategy (assay A) was based on a colorimetric and luminometric assay using the cell line of genetically modified reporter monocytes THP1-Dual^{™} to analyze the activation of two key inflammatory pathways such as the interferon stimulated response element (IRSE) and NF-κB-activated promoters, respectively (Figure 18C). Taking advantage of the fact that the morphological response of microglia is closely related to their degree of activation and inflammatory response, assay B (Figure 18D) used the human microglia line HMC3, the morphological responses of which were evaluated automatically by means of artificial intelligence algorithms such as Cellpose and in a Python workflow. These results, together with neurocognitive analyses, allowed insight into the patients' condition(Figure 18E).

The proposed multicenter validation and strategic commercial partnership (Figure 18F) underline the projections of the present work.

### 2. Detailed methodology

Patient recruitment and neurocognitive tests. Blood samples were taken from men and women ≥ 60 years of age (no upper limit) who sought advice for MCI and/or suspected iAD. Informed consent was obtained from participants according to the guidelines of the accredited bioethics committee of Universidad de Los Andes. Illiterate individuals, with decompensated medical pathologies and psychiatric diseases, were excluded from this study. This recruitment was conducted mainly from the Community Mental Health Center (COSAM) of La Reina and from Clinica Universidad de los Andes. For the recruitment of control patients, samples were obtained from volunteers who were informed about the study through posters and digital platforms of Clinica Universidad de los Andes, Center for Research and Biomedical Innovation (CIIB) and COSAM of La Reina. The initial clinical diagnosis of patients was established by means of clinical assessment with the following neurocognitive tests:
1. Montreal Cognitive Assessment adapted for Chile (MoCa S1-2) to ensure normal general cognitive function.
2. Yesavage, to rule out depression, generating a geriatric depression scale (GDS).
3. Grober and Buschke verbal episodic memory test to evaluate MCI iAD subtype (amnestic or non-amnestic).
4. INECO test to evaluate executive functions in order to rule out or suggest another type of alterations such as frontotemporal dementia (FTD) or others of similar pathogenesis.
5. T-ADLQ questionnaire, to assess maintenance of daily activities, which is a key criterion to diagnose/differentiate iAD from MCI subtypes.
6. Use of DSM V criteria to confirm the absence of dementia.
7. Frontal assessment battery to rule out or suggest another type of alterations such as frontotemporal dementia (FTD) or others of similar pathogenesis.

Based on these instruments, the CDR (clinical dementia rate) values for controls were expected to be CDR=0, while patients with deterioration were classified with a CDR=0.5 or higher. This classification was complemented by means of the global deterioration scale or GDS. Arrangement was made for the accepted study subjects to go to the clinic for the informed consent and evaluation process, which included a 15-minute interview with neurologists and an additional evaluation by the neuropsychologist using the same instruments described above. The total estimated time for this process was 120 minutes. For sample taking and EV isolation, 8 ml of blood was collected in tubes with EDTA anticoagulant. The samples were transferred to the neuroscience laboratory to obtain cell-free plasma according to established protocols.

### 3. Obtaining EVs

EVs were isolated from 0.5 ml of plasma samples isolated from patients using a commercial kit (Invitrogen, 4478359). These EVs were characterized by established methods in the laboratory, which included protein quantification by BCA method and particle number and size quantification by Nanosight.

### 4. Cultures and stimulation with EVs from patients

Human microglia cell line (HMC3) and human reporter monocyte cell line (THP1-Dual^{™}) were cultured in parallel in individual 96-well plates, seeded at a density of 5000 cells per well. Media and antibiotics corresponding to each cell line were used according to literature. THP1-Dual monocytes were used to measure the activation of NF-kB activation (a transcription factor that regulates proinflammatory genes) and interferon stimulated response elements IRSE. After 48 hours in culture, these lines were incubated with 12 micrograms (HMC3) or 120 micrograms (THP-1 dual) of plasma EVs from WI, a iAD, na iAD, a MCI and na MCI patients for 24 hours. Then, 'sensing microglia' was fixed and stained with a broad fluorescent cell contour marker (Cellmask) and DAPI was used for nuclei. For the monocyte assay, in the last 4 hours of stimulation with EVs, cells were treated with 100 ng/mL of *E. coli* LPS (sc-221855), furthermore a control group was stimulated only with LPS respecting the concentration and timing of the rest (baseline condition).

### 5. Colorimetric and luminometric analysis

Once incubated with vesicles from patients, THP1-Dual^{™} reporter cells were treated according to the protocol indicated by the company to measure alkaline phosphatase enzyme activity under the control of NF-kB promoter or luciferase activity (luminometric) for IRSE.

### 6. Image acquisition and analysis

Treated microglia was captured by means of high-resolution microscopy with Cell Observer equipment in collaboration with REDECA (University of Chile). Images were segmented (i.e., cell contours were traced) using the free, artificial intelligence-based software Cellpose. Subsequently, the reported morphometric parameters were analyzed using the algorithms of Fiji/Image J software (Wayne Rasband, NIH, USA) in an automated analysis flow by means of a Python 3 program, specially designed by the present team of engineers at Universidad de Los Andes.

### 7. Statistical analysis of THP-1 and images

For colorimetric and luminometric measurements of THP1-Dual^{™} reporter cells, the average of the observations per patient of each group was calculated and fold changes with respect to the baseline response of the cells to LPS (without treatment with EVs) were calculated. Furthermore, fold changes for the NF-kB pathway were normalized for each patient by fold changes for the IRSE pathway according to this same scheme. The predictive model for reporter cells was evaluated by constructing an ROC curve with the ratios of change of inflammatory pathway activation in THP-1 and with selected morphometric parameters in HMC3 cells.

### Results

### 1) Characterization of patient-derived extracellular plasma vesicles

Once the patients were recruited, plasma EVs were then isolated and characterized in terms of quantity and size by means of nano tracking particle analysis (NTA, with Nanosight equipment). The particle size of this fraction is compatible with that previously reported in the literature where most of them are between 100 and 400 nm in size (results not shown). When the total amount of vesicles present in 1 mL of plasma from each patient was evaluated, it was found that in the WI condition the average number of particles is about half that in MCI and one third in the iAD condition, where this difference between WI and iAD was statistically significant (Figure 19, left). Furthermore, this measurement was compared within the amnestic and non-amnestic subtypes of MCI and iAD. Interestingly, a trend that the average number of particles present in the amnestic conditions is higher than in the non-amnestic conditions is observed in both MCI and iAD (Figure 19, middle panel). Moreover, patients' score in the MOCA cognitive test correlates negatively with the total number of particles present in the patient-derived EV sample (Figure 19, right). These results are compatible with the literature in which it has been observed that MCI and iAD patients tend to have more plasma EVs than controls, but furthermore, they add a novel datum, suggesting that the presence of amnestic-type impairment contributes more to these differences.

### 2) Diagnosis by means of the THP1-Dual^{™} cellular sensor

As a first approach to differentiate between patient-derived extracellular vesicles (EVs), the cellular sensor concept with a cell line of genetically modified monocytes (macrophage precursor) (THP1-Dual^{™}) is used to report inflammation. In this system, the NF-κB promoter is inserted upstream of a sequence encoding an alkaline phosphatase (SEAP) that will be secreted into the extracellular medium. In this way, it is possible to associate a colorimetric reaction with the degradation of its substrate. Furthermore, this line stably expresses secreted luciferase (Lucia) under the control of the interferon stimulated response element (IRSE). The sensing monocytes are incubated directly with patient-derived EVs for 24 hours and their activity is measured. It is found that samples from control patients tend to cluster at slightly higher values, although the differences are not statistically significant (Figure 20A, left panel).

By means of a complementary strategy, the effect of patient-derived EVs on the monocyte inflammatory response to an LPS (lipopolysaccharide) challenge is evaluated. This strategy successfully revealed statistically significant differences between the WI group compared to the MCI and iAD groups (Figure 19A, middle panel). Moreover, this approach achieved very high sensitivity and specificity with an area under the ROC curve of 0.91 (WI vs. MIC) and 0.97 (WI vs. iAD) (Figure 20A, right panels), although it failed to differentiate between subtypes of each condition. This was measured as fold changes of each condition with respect to pathway activation by LPS in cells that were not treated with the vesicles. Based on this same LPS response strategy, a ratio of NF-κB pathway activation to IRSE pathway activation is measured. Interestingly, it is found that this ratio tends to better discriminate between iAD subtypes by comparison to WI, thus the area under the ROC curve was 0.55 to differentiate between WI and na iAD, but for WI and a iAD it was 0.73 (Figure 19B). Although these results are preliminary results, they open the possibility that the ratio of the NF-κB/IRSE pathway response to LPS may contribute to guide the differential diagnosis between iAD subtypes.

### 3) Diagnosis by means of morphometric analysis of human microglia HMC3

To understand the morphological changes triggered by patient-derived EVs in microglia, they are first skeletonized in an automated manner which consists of collapsing all their projections to the minimum central value ("skeleton" formation). From this filamentous structure, various parameters as indicated in Figure 19C are measured. The branching point and termination point parameters are quantified, generating statistically significant differences between the means of na MCI and a iAD, as well as between a MCI and a iAD (Figure 19D, both graphs). Notably, it is observed that each of these parameters has identical capacity to discriminate between WI and a iAD (AUC= 0.78) and between na MCI and na iAD (AUC= 0.77) (Figure 19E). Furthermore, both morphological parameters contribute to differentiate between the a MCI and a iAD groups with AUC greater than or equal to 0.88 (Figure 19E).

Understanding that the stage of mild cognitive impairment may be the prelude to dementia, this strategy would be sensitive to the severity of the impairment within each amnestic and non-amnestic subtype. However, this strategy did not allow finding differences between amnestic and non-amnestic subtypes within MCI or iAD groups.

Finally, the capacity for discriminating the different morphological variables by means of ROC curves is evaluated, highlighting a value of 0.9 for the roundness parameter by comparing WI patients with na iAD patients (Figure 2F). Interestingly, other morphometric parameters such as cell circularity allowed differentiating between na iAD and a iAD, reaching an AUC of 0.79, highlighting the potential to identify initial dementia subtypes. All this preliminary background strongly supports the idea that a combination of these cellular sensor-based strategies could: i) show whether or not the patient has any neurocognitive impairment (by means of THP-1 cells), ii) quantify the intensity of the amnestic or non-amnestic pathology, i.e., determine if the patient is an MCI or iAD patient within one and the same subtype (by means of skeletonization), iii) differentiate between the amnestic and non-amnestic subtypes within the iAD condition (for example, by means of the circularity parameter).

### Conclusions

This study preliminarily introduces two complementary approaches using the cellular sensor concept to diagnose MCI and iAD. The first strategy is based on a colorimetric and luminometric assay using the THP1-Dual^{™} reporter monocyte cell line, which has revealed marked and significant differences between healthy controls and subjects with cognitive impairment or early dementia.

However, although effective in confirming the presence of a pathological condition, this test alone does not distinguish between cognitive impairment or dementia subtypes. In contrast, the second strategy focuses on morphological changes in the HMC3 microglial cell line as a sensor, which could not only confirm the presence of iAD, but also help to differentiate between its subtypes, specifically amnestic and non-amnestic early dementia.

### References

[1] M. First, "Introducción a las enfermedades mentales - Trastornos de la salud mental", MSD Manual, consumer version. Accessed on: 5 January 2024. [Online]. Available at: https://www.msdmanuals.com/es-cl/hogar/trastornos-de-la-salud-men tal/introducci%C3%B3n-al-cuidado-de-la-salud-mental/introducci%C3%B3n-a-las-enfermedades-mentales
[2] American Psychiatric Association, DSM-5 Task Force. (2013). Diagnostic and statistical manual of mental disorders: DSM-5™ (5th ed.). American Psychiatric Publishing, Inc. https://doi.org/10.1176/appi.books.9780890425596.
[3] S. A. Codeluppi et al., "Chronic Stress Alters Astrocyte Morphology in Mouse Prefrontal Cortex", International Journal of Neuropsychopharmacology, vol. 24, no. 10, pages 842-853, October 2021, doi: 10.1093/ijnp/pyab052.
[4] B. Vásquez, M. del Sol, "Morfología Neuronal en el Trastorno del Espectro Autista", International Journal of Morphology, vol. 38, no. 5, pages 1513-1518, October 2020, doi: 10.4067/S0717-95022020000501513.
[5] "Cell Types & Culture Characteristics". Accessed on: 15 July 2024. [Online]. Available at: https://www.sigmaaldrich.com/CL/es/technical-documents/technicalarticle/cell-culture-and-cell-culture-analysis/mammalian-cell-culture/cell-types-culture
[6] "Global, regional, and national burden of 12 mental disorders in 204 countries and territories, 1990-2019: a systematic analysis for the Global Burden of Disease Study 2019", The Lancet Psychiatry, vol. 9, no. 2, pages 137-150, February 2022, doi: 10.1016/S22150366(21)00395-3.
[7] S. H. Kennedy, "Core symptoms of major depressive disorder: relevance to diagnosis and treatment", Dialogues Clin Neurosci, vol. 10, no. 3, pages 271-277, 2008, doi: 10.31887/DCNS.2008.10.3/shkennedy
[8] B. N. Frey et al., "The Early Burden of Disability in Individuals With Mood and Other Common Mental Disorders in Ontario, Canada", JAMA Network Open, vol. 3, no. 10, page e2020213, October 2020, doi: 10.1001/jamanetworkopen.2020.20213.
[9] J. Flint and K. S. Kendler, "The genetics of major depression", Neuron, vol. 81, no. 3, pages 484-503, February 2014, doi: 10.1016/j.neuron.2014.01.027.
[10] C. J. Murray and A. D. Lopez, "Evidence-based health policy-lessons from the Global Burden of Disease Study", Science, vol. 274, no. 5288, pages 740-743, November 1996, doi: 10.1126/science.274.5288.740.
[11] R. M. A. Hirschfeld, L. Lewis, and L. A. Vornik, "Perceptions and impact of bipolar disorder: how far have we really come? Results of the national depressive and manic-depressive association 2000 survey of individuals with bipolar disorder", J Clin Psychiatry, vol. 64, no. 2, pages 161-174, February 2003, PMID: 12633125.
[12] M. Colombo, G. Raposo, and C. Théry, "Biogenesis, secretion, and intercellular interactions of exosomes and other extracellular vesicles", Annu Rev Cell Dev Biol, vol. 30, pages 255- 289, 2014, doi: 10.1146/annurev-cellbio-101512-122326.
[13] S. Keerthikumar et al., "ExoCarta: A Web-Based Compendium of Exosomal Cargo", J Mol Biol, vol. 428, no. 4, pages 688-692, February 2016, doi: 10.1016/j.jmb.2015.09.019.
[14] M. Quaak, L. van de Mortel, R. M. Thomas, and G. van Wingen, "Deep learning applications for the classification of psychiatric disorders using neuroimaging data: Systematic review and meta-analysis", Neurolmage: Clinical, vol. 30, page 102584, 2021, doi: https://doi.org/10.1016/j.nicl.2021.102584.
[15] R. S. Kumar, A. Singh, S. Srinath, N. K. Thomas, and V. Arasu, "Skin Cancer Detection using Deep Learning", 2022 International Conference on Electronics and Renewable Systems (ICEARS), Match 2022, pages 1724-1730. doi: 10.1109/ICEARS53579.2022.9751826.
[16] S. Swaroop, S. Sharma, and Janarthanan. S, "Lung Cancer Classification and Through Deep Learning Model and Localization of Tumor", 2023 9th International Conference on Advanced Computing and Communication Systems (ICACCS), March 2023, pages 1530-1534. doi: 10.1109/ICACCS57279.2023.10112822.
[17] R. Morelli et al., "Automating cell counting in fluorescent microscopy through deep learning with c-ResUnet", Sci Rep, vol. 11, p. 22920, November 2021, doi: 10.1038/s41598021-01929-5.
[18] G. Alwakid, W. Gouda, M. Humayun, and N. Z. Jhanjhi, "Diagnosing Melanomas in Dermoscopy Images Using Deep Learning", Diagnostics (Basel), vol. 13, no. 10, page 1815, May 2023, doi: 10.3390/diagnostics13101815.
[19] G. Ayano et al., "Misdiagnosis, detection rate, and associated factors of severe psychiatric disorders in specialized psychiatry centers in Ethiopia", Ann Gen Psychiatry, vol. 20, no. 1, p. 10, February 2021, doi: 10.1186/s12991-021-00333-7.
[20] N. Bains and S. Abdijadid, "Major Depressive Disorder", StatPearls, Treasure Island (FL): StatPearls Publishing, 2023. Accessed on: 16 January 2024. [Online]. Available at: http://www.ncbi.nlm.nih.gov/books/NBK559078/. PMID: 32644504.
[21] "What Are Bipolar Disorders?" Accessed on: 16 January 2024. [Online]. Available at: https://www.psychiatry.org:443/patients-families/bipolar-disorders/what-arebipolar-disorders
[22] D. J. Stein, K. A. Phillips, D. Bolton, K. W. M. Fulford, J. Z. Sadler, and K. S. Kendler, "What is a Mental/Psychiatric Disorder? From DSM-IV to DSM-V", Psychol Med, vol. 40, no. 11, pages 1759-1765, November 2010, doi: 10.1017/S0033291709992261.
[23] "Bipolar Disorder - National Institute of Mental Health (NIMH)". Accessed on: 16 January 2024. [Online]. Available at: https://www.nimh.nih.gov/health/publications/bipolar-disorder
[24] R. M. Ransohoff and J. E. Khoury, "Microglia in Health and Disease", Cold Spring Harb Perspect Biol, vol. 8, no. 1, page a020560, January 2016, doi: 10.1101/cshperspect.a020560.
[25] D. Bella and M. Antonietta, "Overview and Update on Extracellular Vesicles: Considerations on Exosomes and Their Application in Modern Medicine", Biology, vol. 11, no. 6, Art. no. 6, June 2022, doi: 10.3390/biology11060804.
[26] "¿Qué es el plasma?", Centro de Transfusión. Accessed on: 23 July 2024. [Online]. Available at: https://www.comunidad.madrid/hospital/centrodetransfusion/ciudadanos/es-plasma
[27] S. R. Sergent and J. V. Ashurst, "Plasmapheresis", StatPearls, Treasure Island (FL): StatPearls Publishing, 2024. Accessed on: 22 July 2024. [Online]. Available at: http://www.ncbi.nlm.nih.gov/books/NBK560566/
[28] R. Paniagua, M. Nistal, P. Sesma, M. Álvarez-Uría, B. Fraile, R. Anadón, and F. Saéz (Eds.), "Biología celular y molecular", 4th ed. McGraw-Hill Education, 2017.
[29] J. M. Helm et al., "Machine Learning and Artificial Intelligence: Definitions, Applications, and Future Directions", Curr Rev Musculoskelet Med, vol. 13, no. 1, pages 69-76, February 2020, doi: 10.1007/s12178-020-09600-8.
[30] T. Amaratunga, "What Is Deep Learning?", Deep Learning on Windows: Building Deep Learning Computer Vision Systems on Microsoft Windows, T. Amaratunga, Ed., Berkeley, CA: Apress, 2021, pages 1-14. doi: 10.1007/978-1-4842-6431-7_1.
[31] A. Krizhevsky, I. Sutskever, and G. E. Hinton, Imagenet classification with deep convolutional neural networks in Proceedings of the 25th International Conference on Neural Information Processing Systems (NIPS), pages 1097-1105, 2012, doi:https://doi.org/10.1145/3065386.
[32] K. He, X. Zhang, S. Ren, and J. Sun, "Deep Residual Learning for Image Recognition". arXiv, 10 December 2015. Accessed on: 20 January 2024. [Online]. Available at: http://arxiv.org/abs/1512.03385, doi:https://doi.org/10.48550/arXiv.1512.03385.
[33] J. Mathew, P. Sankar, and M. Varacallo, "Physiology, Blood Plasma", StatPearls, Treasure Island (FL): StatPearls Publishing, 2023. Accessed on: 20 January 2024. [Online]. Available at: http://www.ncbi.nlm.nih.gov/books/NBK531504/
[34] I. Nassiri and M. N. McCall, "Systematic exploration of cell morphological phenotypes associated with a transcriptomic query", Nucleic Acids Res, vol. 46, no. 19, page e116, November 2018, doi: 10.1093/nar/gky626.
[35] "Transfer Learning". Accessed on: 23 January 2024. [Online]. Available at: https://la.mathworks.com/discovery/transfer-learning.html
[36] "What are Convolutional Neural Networks? | IBM". Accessed on: 23 January 2024. [Online]. Available at: https://www.ibm.com/topics/convolutional-neural-networks
[37] "¿Qué es una red neuronal? | IBM". Accessed on: 22 July 2024. [Online]. Available at: https://www.ibm.com/es-es/topics/neural-networks
[38] H. Zheng et al., "Learn From Model Beyond Fine-Tuning: A Survey". arXiv, 12 October 2023. Accessed on: 2 July 2024. [Online]. Available at: http://arxiv.org/abs/2310.08184, doi: https://doi.org/10.48550/arXiv.2310.08184.
[39] I. Goodfellow, Y. Bengio, and A. Courville, "Deep Learning". MIT Press, 2016. [Online]. Available: http://www.deeplearningbook.org
[40] B. Gunter, Media Research Methods. SAGE Publications, Ltd, 2000. doi: 10.4135/9780857028983.
[41] N. Srivastava, G. Hinton, A. Krizhevsky, I. Sutskever and R. Salakhutdinov, "Dropout: A Simple Way to Prevent Neural Networks from Overfitting", J. Mach. Learn. Res., Vol. 15, No. 1, pages 1929-158, 2014.
[42] M. Oquab, L. Bottou, I. Laptev and J. Sivic, "Learning and Transferring Mid-level Image Representations Using Convolutional Neural Networks", 2014 IEEE Conference on Computer Vision and Pattern Recognition, pages 1717-1724, 2014, doi:10.1109/CVPR.2014.222.
[43] J. Yosinski, J. Clune, Y. Bengio and H. Lipson, "How Transferable Are Features in Deep Neural Networks?", Proceedings of the 27th International Conference on Neural Information Processing Systems - Volume 2, ser. NIPS'14. Cambridge, MA, USA: MIT Press, 2014, doi: https://doi.org/10.48550/arXiv.1411.1792
[44] J. Latif, C. Xiao, A. Imran, and S. Tu, "Medical Imaging using Machine Learning and Deep Learning Algorithms: A Review", 2019 2nd International Conference on Computing, Mathematics and Engineering Technologies (iCoMET), January 2019, pages 1-5. doi: 10.1109/ICOMET.2019.8673502.
[45] T. Ching et al., "Opportunities and obstacles for deep learning in biology and medicine", Journal of The Royal Society Interface, vol. 15, no. 141, p. 20170387, April 2018, doi: 10.1098/rsif.2017.0387.
[46] F. Piccialli, V. D. Somma, F. Giampaolo, S. Cuomo, and G. Fortino, "A survey on deep learning in medicine: Why, how and when?", Information Fusion, vol. 66, pages 111-137, February 2021, doi: 10.1016/j.inffus.2020.09.006.
[47] D. Shen, G. Wu, and H. I. Suk, "Deep Learning in Medical Image Analysis", Annual Review of Biomedical Engineering, vol. 19, volume no. 19, 2017, pages 221-248, June 2017, doi: 10.1146/annurev-bioeng-071516-044442.
[48] J. Ker, L. Wang, J. Rao, and T. Lim, "Deep Learning Applications in Medical Image Analysis", IEEE Access, vol. 6, pages 9375-9389, 2018, doi: 10.1109/ACCESS.2017.2788044.

## Claims

1. A method for identifying the risk of a human subject having or suffering from an age-related disease or a mood disorder, wherein the method comprises the following steps:
a. exposing specific human immune cells of the central nervous system, preferably microglial cells, *in vitro* to i) a biological sample isolated from the subject which comprises extracellular vesicles and/or ii) a composition which comprises extracellular vesicles isolated from the subject, for a given time, preferably for 1 hour and 72 hours, more preferably for 12 hours and 36 hours, more preferably for about 24 hours;
b. analyzing morphological changes in the cultured microglial cells; and
c. determining the risk of the subject having or suffering from an age-related disease or a mood disorder using a convolutional neural network (CNN) trained to recognize morphological patterns indicative of the disease.

2. The method according to claim 1, wherein the method identifies the risk of a subject having or suffering from an age-related disease selected from the list consisting of cognitive impairment or dementia.

3. The method according to claim 1, wherein the method identifies the risk of a subject having or suffering from a mood disorder selected from the list consisting of MDD (major depressive disorder) or BD (bipolarity or bipolar disorder (BP for bipolar disorder)).

4. The method according to claim 1, wherein the method identifies the risk of a subject having or suffering from mild cognitive impairment, preferably amnestic mild cognitive impairment or non-amnestic mild cognitive impairment.

5. The method according to claim 1, wherein the method identifies the risk of a subject having or suffering from initial amnestic dementia, preferably initial amnestic dementia or non-amnestic initial amnestic dementia.

6. The method according to claim 1, wherein the method distinguishes between subjects having or suffering from MCI (mild cognitive impairment) and iAD (initial amnestic dementia), or between subjects having or suffering from na MCI (non-amnestic mild cognitive impairment) and iAD, or between subjects having or suffering from na iAD (initial non-amnestic dementia) and a iAD (initial amnestic dementia).

7. The method according to any of claims 1 to 6, wherein the CNN comprises a ResNet18 or ResNet50 architecture.

8. The method according to any of claims 1 to 7, wherein the CNN is trained with a dataset of labelled microglial cell morphology images related to the disease and control samples.

9. The method according to any of claims 1 to 8, wherein morphological changes are determined by measuring parameters selected from the group consisting of cell shape, size, granularity, motility, clustering and/or distribution of intracellular organelles.

10. The method according to any of claims 7 to 9, wherein the ResNet architecture is pre-trained in a general dataset of images and specifically fitted using a labelled dataset related to the morphological patterns of microglial cells exposed to biological samples from subjects with the disease.

11. The method according to any of claims 1 to 10, wherein the biological sample is a blood plasma sample from the subject.

12. The method according to any of claims 1 to 11, wherein the microglial cells are HMC3 cells.

13. The method according to any of claims 1 to 12, wherein the determination of morphological changes comprises high content imaging and automated processing of images to generate quantitative data for analysis by means of the ResNet architecture.

14. A system configured to implement the method according to any of claims 1 to 13, wherein the system comprises:
a. an image acquisition module configured to capture high-resolution images of microglial cells exposed to the biological sample or a composition comprising extracellular vesicles;
b. an image processing module configured to analyze morphological changes in the images obtained;
c. an artificial intelligence module which uses a convolutional neural network, preferably based on a ResNet18 or ResNet50 architecture, to classify the morphological changes;
d. a result generation module configured to provide the user with a score of the risk or likelihood of the subject having or developing the disease, and/or optionally a graphic display of the results; and
e. a user interface which allows displaying and downloading the results.

15. A computer program stored in a computer-readable medium which, when executed in a computer system, is configured to perform a method for identifying the risk of a subject having or suffering from a disease according to any of claims 1 to 13, wherein the program comprises the following instructions:
a. receiving images of microglial cells exposed to a biological sample or a composition comprising extracellular vesicles;
b. processing the received images to detect and quantify morphological changes in the cells;
c. applying a convolutional neural network, preferably based on a ResNet18 or ResNet50 architecture, trained to classify morphological data and to generate a risk score related to the disease; and
d. providing the result of the analysis to the user, preferably by means of a graphic interface or through a cloud-based platform.
